# EUROPEAN PATENT APPLICATION

(11) **EP 3 669 958 A1**
(43) Date of publication of application: **24.06.2020**
(21) Application number: 18847028.0
(22) Date of filing: 31.07.2018
(51) Int. Cl.: A63J 5/02

(54) **INFORMATION PROCESSING DEVICE, INFORMATION PROCESSING METHOD, AND PROGRAM**

(30) Priority: 14.08.2017 JP 2017156551
(71) Applicant: Sony Corporation, Tokyo 108-0075 (JP)
(72) Inventor: KIMURA Takaomi, Tokyo 108-0075 (JP)
(74) Representative: MFG Patentanwälte Meyer-Wildhagen Meggle-Freund Gerhard PartG mbB
(86) International application number: PCT/JP2018/028554
(87) International publication number: WO 2019/035350

(57) **Abstract**

The present disclosure relates to an information processor, an information processing method, and a program that make it possible to easily and accurately recognize a motion of an object.

The information processor includes: a motion recognition section that recognizes a motion of an object on the basis of a plurality of detection data indicating respective movements of a plurality of detection positions of the object detected by a plurality of sensor apparatuses at the plurality of detection positions; and an output control section that controls an output of control data used for controlling of processing corresponding to the motion of the object. The present technology is applicable to, for example, a rendering system that performs rendering of a performance.

## Description

### Technical Field

The present disclosure relates to an information processor, an information processing method, and a program, and more particularly, to an information processor, an information processing method, and a program suitable for use in a case of recognizing a motion of an object.

### Background Art

There has been proposed a system in which, on the basis of exercise information from sensors attached to a plurality of users, a motion-related arithmetic unit estimates an exercise rhythm of each user, calculates a degree of difference between the exercise rhythm of each user and a basic exercise rhythm, or a degree of difference between the exercise rhythms of the users, and feeds back results thereof to the user (see, e.g., PTL 1).

### Citation List

### Patent Literature

PTL 1: Japanese Unexamined Patent Application Publication No. 2011-87794

### Summary of the Invention

### Problems to be Solved by the Invention

However, in the invention described in PTL 1, it is not considered to recognize, for example, a specific motion such as a dance technique of each user.

The present technology has been made in view of such a circumstance, and makes it possible to easily and accurately recognize a motion of an object such as a person.

### Means for Solving the Problem

An information processor according to an aspect of the present technology includes a required time estimation section that estimates first required time required for communication data to arrive at an apparatus in a subsequent stage, and a transmission section that transmits the communication data and required time information including the first required time to the apparatus in the subsequent stage.

In an information processing method according to an aspect of the present technology, the information processor recognizes a motion of an object on the basis of a plurality of detection data indicating respective movements of a plurality of detection positions of the object detected by a plurality of sensor apparatuses at the plurality of detection positions, and controls an output of control data used for controlling of processing corresponding to the motion of the object.

A program according to an aspect of the present technology causes a computer to execute processing including a motion recognition step that recognizes a motion of an object on the basis of a plurality of detection data indicating respective movements of a plurality of detection positions of the object detected by a plurality of sensor apparatuses at the plurality of detection positions, and an output control step that controls an output of control data used for controlling of processing corresponding to the motion of the object.

In an aspect of the present technology, a motion of an object is recognized on the basis of a plurality of detection data indicating respective movements of a plurality of detection positions of the object detected by a plurality of sensor apparatuses at the plurality of detection positions, and an output of control data used for controlling of processing corresponding to the motion of the object is controlled.

### Effect of Invention

According to an aspect of the present technology, it is possible to recognize the motion of an object such as a person easily and accurately.

It is to be noted that the effects described herein are not necessarily limited, and may be any of the effects described in the present disclosure.

### Brief Description of the Drawings

[FIG. 1] FIG. 1 is a block diagram illustrating a first embodiment of a motion recognition system to which the present technology is applied.
[FIG. 2] FIG. 2 illustrates an example of attaching positions of a wearable sensor.
[FIG. 3] FIG. 3 is a block diagram illustrating a first embodiment of the wearable sensor.
[FIG. 4] FIG. 4 is a table illustrating an example of specifications of sensors provided in the wearable sensor.
[FIG. 5] FIG. 5 is a block diagram illustrating a first embodiment of a motion recognizer.
[FIG. 6] FIG. 6 is an explanatory flowchart of a first embodiment of processing of the wearable sensor.
[FIG. 7] FIG. 7 illustrates a first embodiment of a format of a communication packet.
[FIG. 8] FIG. 8 is a table illustrating a configuration example of motion detection data.
[FIG. 9] FIG. 9 is an explanatory flowchart of a first embodiment of processing of the motion recognizer.
[FIG. 10] FIG. 10 illustrates an example of a case of performing motion recognition in figure skating.
[FIG. 11] FIG. 11 illustrates examples of dance techniques
[FIG. 12] FIG. 12 illustrates examples of dance techniques.
[FIG. 13] FIG. 13 is a block diagram illustrating a configuration example of a learning system to which the present technology is applied.
[FIG. 14] FIG. 14 is a block diagram illustrating a configuration example of an information processor.
[FIG. 15] FIG. 15 is a block diagram illustrating a configuration example of a learning processing unit.
[FIG. 16] FIG. 16 is an explanatory flowchart of motion registration processing.
[FIG. 17] FIG. 17 illustrates an example of user interfaces of the motion registration processing.
[FIG. 18] FIG. 18 illustrates hierarchical structure of the genre of dances.
[FIG. 19] FIG. 19 illustrates hierarchical structure of dance techniques.
[FIG. 20] FIG. 20 illustrates examples of learning data.
[FIG. 21] FIG. 21 is a graph illustrating a relationship between data loss rate and motion recognition accuracy.
[FIG. 22] FIG. 22 is a table illustrating a relationship among the number of retransmissions of communication packets, data loss rate, maximum delay time, and motion recognition accuracy.
[FIG. 23] FIG. 23 is an explanatory diagram of elements contributing to a real-time property of motion recognition.
[FIG. 24] FIG. 24 is a block diagram illustrating a second embodiment of the motion recognition system to which the present technology is applied.
[FIG. 25] FIG. 25 is a block diagram illustrating a second embodiment of the wearable sensor.
[FIG. 26] FIG. 26 is a block diagram illustrating a second embodiment of the motion recognizer.
[FIG. 27] FIG. 27 is an explanatory flowchart of a second embodiment of the processing of the motion recognizer.
[FIG. 28] FIG. 28 is an explanatory flowchart of a second embodiment of the processing of the wearable sensor.
[FIG. 29] FIG. 29 illustrates a second embodiment of the format of a communication packet.
[FIG. 30] FIG. 30 is a sequence diagram illustrating communication between the motion recognizer and each of wearable sensors.
[FIG. 31] FIG. 31 is a block diagram illustrating a third embodiment of the motion recognition system to which the present technology is applied.
[FIG. 32] FIG. 32 is a block diagram illustrating a third embodiment of the wearable sensor.
[FIG. 33] FIG. 33 is a block diagram illustrating a third embodiment of the motion recognizer.
[FIG. 34] FIG. 34 is an explanatory flowchart of a third embodiment of the processing of the wearable sensor.
[FIG. 35] FIG. 35 illustrates a third embodiment of the format of a communication packet.
[FIG. 36] FIG. 36 is an explanatory flowchart of a third embodiment of the processing of the motion recognizer.
[FIG. 37] FIG. 37 is a block diagram illustrating a fourth embodiment of the motion recognition system to which the present technology is applied.
[FIG. 38] FIG. 38 is a block diagram illustrating a configuration example of a relay.
[FIG. 39] FIG. 39 is an explanatory flowchart of processing of the relay.
[FIG. 40] FIG. 40 illustrates a fourth embodiment of the format of a communication packet.
[FIG. 41] FIG. 41 is a block diagram illustrating a configuration example of a rendering system to which the present technology is applied.
[FIG. 42] FIG. 42 is a block diagram illustrating a configuration example of a rendering control unit.
[FIG. 43] FIG. 43 is an explanatory flowchart of rendering control processing.
[FIG. 44] FIG. 44 is a transition diagram illustrating a relationship between motion transition and sound effect.
[FIG. 45] FIG. 45 illustrates examples of timings at which sound effect is outputted.
[FIG. 46] FIG. 46 is a block diagram illustrating a configuration example of an analysis system to which the present technology is applied.
[FIG. 47] FIG. 47 is a block diagram illustrating a configuration example of an analysis processing unit.
[FIG. 48] FIG. 48 is an explanatory flowchart of analysis processing.
[FIG. 49] FIG. 49 illustrates a first example of a presentation method of the analysis processing.
[FIG. 50] FIG. 50 is an enlarged view of displayed contents of an action analysis part.
[FIG. 51] FIG. 51 illustrates a second example of the presentation method of the analysis processing.
[FIG. 52] FIG. 52 illustrates a third example of the presentation method of the analysis processing. Modes for Carrying Out the Invention

Hereinafter, description is given of embodiments for carrying out the present technology. The description is given in the following order.
1. First Embodiment (Motion Recognition System and Learning System)
2. Second Embodiment (Motion Recognition System)
3. Third Embodiment (Motion Recognition System)
4. Fourth Embodiment (Motion Recognition System)
5. Fifth Embodiment (Rendering System)
6. Sixth Embodiment (Analysis System)
7. Application Example
8. Modification Example
9. Others

### <<1.First Embodiment>>

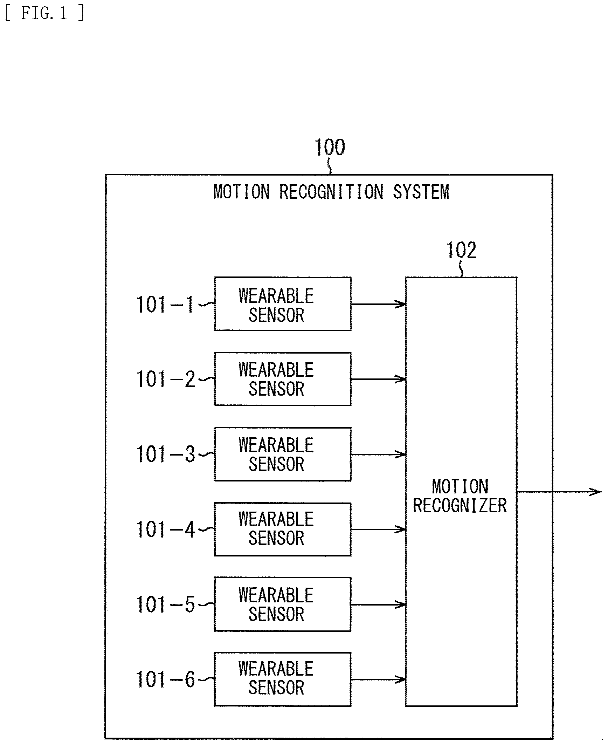

First, with reference to FIGs. 1 to 23, description is given of a first embodiment of the present technology.

### <First Embodiment of Motion Recognition System>

FIG. 1 illustrates a configuration example of a motion recognition system 100 which is a first embodiment of a motion recognition system to which the present technology is applied.

The motion recognition system 100 is a system that performs recognition processing of motion of a user.

Here, the motion refers to, for example, a unit of movements constituting an action of a user (person) on the basis of predetermined classification. Thus, the motion is a user movement in a time period shorter than action. In addition, the motion may be a movement of the whole body of the user or a movement of a portion of the body of the user.

Meanwhile, an action is an aggregation of a series of motions on the basis of predetermined classification, and includes a combination of a plurality of motions. For example, an aggregation of a series of motions for achieving a predetermined purpose is an action.

It is to be noted that, in the present technology, it is possible to arbitrarily set or change the classification of actions and motions. For example, the actions to be subjected to motion recognition are classified on the basis of sports events such as dance, ice skating, rugby, triathlon, swimming, and mountain climbing. In this case, for example, sports in which a plurality of events are combined, such as all-around gymnastics, may be regarded as one action.

Further, for example, an action to be a target of motion recognition is classified on the basis of actions of daily life such as commuting, driving, work, study, raising children, sleeping, cooking, eating, watching TV, singing, playing, photographing, and the like.

Meanwhile, for example, a motion to be recognized is classified on the basis of dance techniques such as slide, jerk, or biz markie, with respect to the action of dancing. Alternatively, the motion to be recognized is classified on the basis of dance movements such as rotation, jump, step, rolling, and pitching, with respect to the action of dancing. It is to be noted that motions to be recognized may be classified on the basis of both techniques and movements of dancing.

For example, a motion to be recognized is classified on the basis of techniques of figure skating such as axel, lutz, or salchow, with respect to an action of figure skating. Alternatively, the motion to be recognized is classified on the basis of the movements of the figure skating, such as sliding, rotating, jumping, and stepping, with respect to the action of the figure skating. It is to be noted that motion to be recognized may be classified on the basis of both techniques and movements of the figure skating.

For example, a motion to be recognized is classified on the basis of rugby-specific movements such as scrum, try, run, pass, and tackle, with respect to the action of rugby. Alternatively, the motion to be recognized is classified on the basis of general motions such as running, jumping, throwing, catching, and hitting, with respect to the action of rugby, for example. It is to be noted that motion to be recognized may be classified on the basis of both rugby-specific movements and the general movements.

For example, a motion to be recognized is classified on the basis of swimming strokes such as crawl, breaststroke, butterfly, and back stroke, with respect to the action of swimming. Alternatively, for example, the motion to be recognized is classified on the basis of the movements of swimming such as jumping-in, turn, breathing, and kick, with respect to the action of swimming. It is to be noted that motions to be recognized may be classified on the basis of both the stroke and the movement of swimming.

For example, a motion to be recognized is classified on the basis of triathlon events of swimming, cycling, and running, with respect to the action of the triathlon.

For example, a motion to be recognized is classified on the basis of events of gymnastics such as vault, floor, and still rings, with respect to the action of the all-around gymnastics.

For example, a motion to be recognized is classified on the basis of movements at the time of commuting such as walking, resting, riding on a train, or climbing a staircase, with respect to the action of commuting.

For example, a motion to be recognized is classified on the basis of movements at the time of driving such as accelerating, braking, gear-changing, and rear confirmation, with respect to the action of driving.

For example, a motion to be recognized is classified on the basis of movements at the time of working, such as speaking, listening, writing, using a telephone or a PC, and discussion, with respect to the action of working.

It is to be noted that the same user's movement may be an action or a motion depending on different classifications. For example, each of the events (swimming, cycling, and running) of the triathlon may be an action to be subjected to motion recognition independently. That is, the swimming, cycling, or running may be classified into actions, and motion recognition processing may be performed for each of the actions.

The motion recognition system 100 includes a wearable sensor 101-1 to a wearable sensor 101-6, and a motion recognizer 102.

It is to be noted that, in the following, in a case where the wearable sensor 101-1 to the wearable sensor 101-6 need not be individually distinguished, they are simply referred to as a wearable sensor 101.

Each wearable sensor 101 is a sensor apparatus that is attached to a different position of a user who is a target of motion recognition (hereinafter, referred to as a detection point or a detection position) and detects a movement of each detection point. Each wearable sensor 101 transmits to the motion recognizer 102 a communication packet including motion detection data indicating motion detection results of the respective detection points.

The motion recognizer 102 is installed at a position remote from the user, for example. Then, the motion recognizer 102 performs processing of motion recognition of the user on the basis of the movements of the detected points of the user detected by the respective wearable sensors 101. The motion recognizer 102 transmits control data used for controlling of processing corresponding to the motion of the user (hereinafter, referred to as motion control data) to an apparatus in a subsequent stage.

It is to be noted that, for communication between the wearable sensor 101 and the motion recognizer 102, for example, low-delay near field communication of any system is adopted. By adopting the near field communication, it is possible to reduce power consumption of the wearable sensor 101 and to miniaturize the wearable sensor 101.

In addition, the communication between the motion recognizer 102 and the apparatus in a subsequent stage adopts, for example, wireless communication or wired communication of any system.

### <Example of Attaching Position of each Wearable Sensor 101>

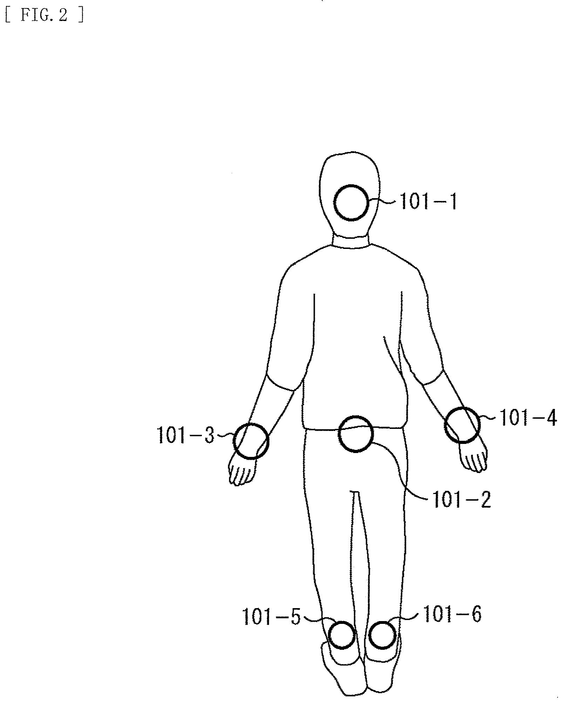

FIG. 2 schematically illustrates examples of attaching positions (detection points) of the wearable sensors 101. It is to be noted that, in the diagram, the attaching positions of the wearable sensor 101 are indicated by circles.

For example, the wearable sensor 101-1 is attached to the head of the user and detects the movement of the head. The wearable sensor 101-2 is attached to the hip of the user and detects the movement of the hip. That is, the wearable sensor 101-1 and the wearable sensor 101-2 detect the movement of body trunk, gravity center, posture, and the like of the user.

The wearable sensor 101-3 is attached to the user's left wrist and detects movement of the left wrist. The wearable sensor 101-4 is attached to the user's right wrist and detects movement of the right wrist. That is, the wearable sensor 101-3 and the wearable sensor 101-4 detect movements of both hands, snaps of wrists, and the like of the user.

The wearable sensor 101-5 is attached to the user's left ankle and detects movement of the left ankle. The wearable sensor 101-6 is attached to the user's right ankle and detects movement of the right ankle. That is, the wearable sensor 101-5 and the wearable sensor 101-6 detect movements, steps, and the like of both feet of the user.

Then, the motion of the user is recognized on the basis of an interlocking property of the mutual movements of the body trunk, both hands, and both feet of the user.

It is to be noted that the number of the wearable sensors 101 attached and the attaching positions thereof in FIGs. 1 and 2 are exemplary, and are appropriately changed depending on the type, etc. of motions to be recognized. For example, the wearable sensor 101 may be attached to two or more of head, torso, left hand, right hand, left foot, and right foot of the user.

It is to be noted that, in general, as the number of the wearable sensors 101 attached increases, motion recognition accuracy increases, while load applied to the user increases. On the other hand, as the number of the wearable sensors 101 attached decreases, the load applied to the user decreases,while the motion recognition accuracy decreases.

In addition, there is no particular limitation on how to attach the wearable sensor 101. For example, a band, a belt, a supporter, a tape, a clip, or the like may be used. It is to be noted that, in order to detect the movement of each of the detection points with high accuracy, it is desirable that the attaching position of each wearable sensor 101 do not move as much as possible to such a degree as not to hinder the movement of the user or to cause a sense of discomfort to the user.

### <Configuration Example of Wearable Sensor 101>

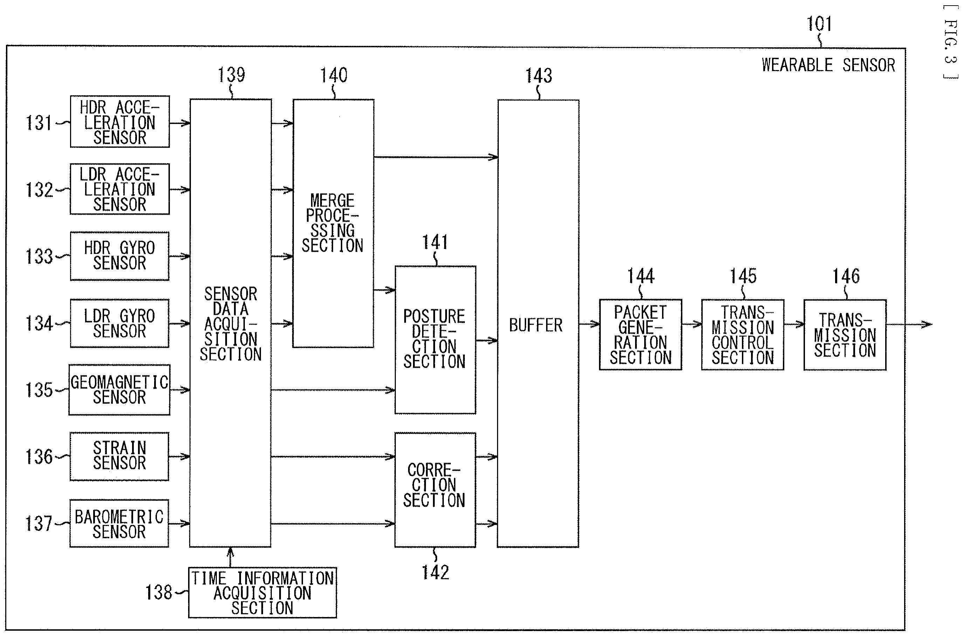

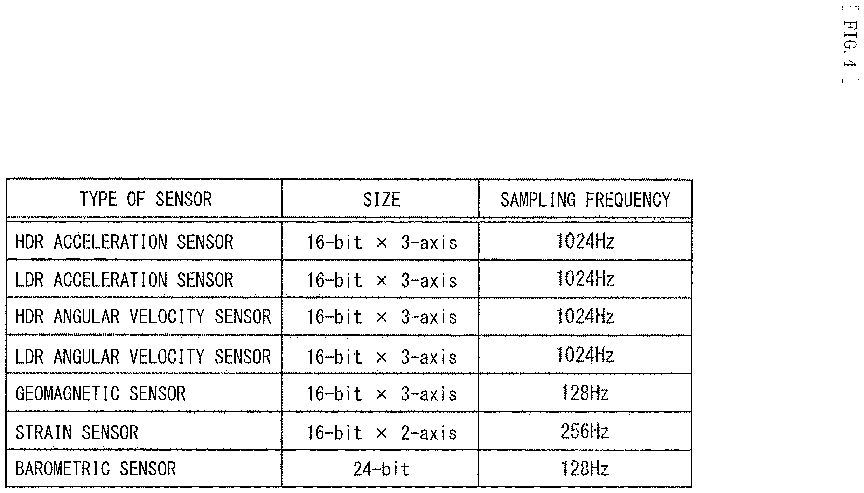

FIG. 3 illustrates a configuration example of the wearable sensor 101 in FIG. 1. In addition, FIG. 4 illustrates examples of specifications of sensors of the wearable sensor 101.

The wearable sensor 101 includes an HDR (High Dynamic Range) acceleration sensor 131, an LDR (Low Dynamic Range) acceleration sensor 132, an HDR (High Dynamic Range) gyro sensor 133, an LDR (Low Dynamic Range) gyro sensor 134, a geomagnetic sensor 135, a strain sensor 136, a barometric sensor 137, a time information acquisition section 138, a sensor data acquisition section 139, a merge processing section 140, a posture detection section 141, a correction section 142, a buffer 143, a packet generation section 144, a transmission control section 145, and a transmission section 146.

The HDR acceleration sensor 131 detects accelerations in three axial directions of a pitch axis (x-axis), a roll axis (y-axis), and a yaw axis (z-axis) at a position (detection point) where the wearable sensor 101 is attached. For example, the HDR acceleration sensor 131 samples accelerations of detection points at a sampling frequency of 1024 Hz, and outputs sensor data of 16 bits×3 axes (hereinafter, referred to as HDR acceleration data).

The LDR acceleration sensor 132 detects acceleration of the detection points in the three axial directions of the pitch axis, the roll axis, and the yaw axis. For example, the LDR acceleration sensor 132 samples accelerations of the detection points at a sampling frequency of 1024 Hz, and outputs sensor data of 16 bits×3 axes (hereinafter, referred to as LDR acceleration data).

It is to be noted that the HDR acceleration sensor 131 has a wider dynamic range of detectable acceleration than that of the LDR acceleration sensor 132. For example, the HDR acceleration sensor 131 outputs sensor data at ±32G, while the LDR acceleration sensor 132 outputs sensor data at ±16G.

The HDR gyro sensor 133 detects an angular velocity of a detection point around three axes, i.e., around the pitch axis, around the roll axis, and around the yaw axis. For example, the HDR gyro sensor 133 samples angular velocities of the detection points at a sampling frequency of 1024 Hz, and outputs sensor data of 16 bits×3 axes (hereinafter, referred to as HDR angular velocity data).

The LDR gyro sensor 134 detects an angular velocity of a detection point around three axes, i.e., around the pitch axis, around the roll axis, and around the yaw axis. For example, the LDR gyro sensor 134 samples angular velocities of the detection points at a sampling frequency of 1024 Hz, and outputs sensor data of 16 bits×3 axes (hereinafter, referred to as LDR angular velocity data).

It is to be noted that the HDR gyro sensor 133 has a wider dynamic range of detectable angular velocity than that of the LDR gyro sensor 134. For example, the HDR gyro sensor 133 outputs sensor data at ±4000 dps, while the LDR gyro sensor 134 outputs sensor data at ±2000 dps.

The geomagnetic sensor 135 detects geomagnetism in the three axial directions of the pitch axis, the roll axis, and the yaw axis near the detection points. For example, the geomagnetic sensor 135 samples the geomagnetism near the detection points at a sampling frequency of 128 Hz, and outputs sensor data of 16 bits×3 axes (hereinafter, referred to as geomagnetism data).

The strain sensor 136 detects strain of the detection point in two axial directions of the pitch axis and the roll axis (e.g., expansion and contraction of the detection point). For example, the strain sensor 136 samples strains of the detection points at a sampling frequency of 256 Hz, and outputs sensor data of 16-bit×2-axis (hereinafter, referred to as strain data).

The barometric sensor 137 detects a barometric pressure near the detection point. For example, the position of the detection point in the height direction is detected on the basis of the barometric pressure near the detection point. For example, the barometric sensor 137 samples barometric pressures near the detection point at a sampling frequency of 128 Hz, and outputs 24-bit sensor data (hereinafter, referred to as barometric pressure data).

The time information acquisition section 138 is configured by, for example, a clock, etc., acquires time information indicating the current time, and supplies the acquired time information to the sensor data acquisition section 139.

The sensor data acquisition section 139 acquires HDR acceleration data, LDR acceleration data, HDR angular velocity data, and LDR angular velocity data from the HDR acceleration sensor 131, the LDR acceleration sensor 132, the HDR gyro sensor 133, and the LDR gyro sensor 134, respectively; adds the acquired time to the acquired data; and supplies the resulting acquired data to the merge processing section 140. Further, the sensor data acquisition section 139 acquires geomagnetic data from the geomagnetic sensor 135, adds the acquired time to the acquired data, and supplies the resulting acquired data to the posture detection section 141. Further, the sensor data acquisition section 139 acquires strain data and barometric pressure data from the strain sensor 136 and the barometric sensor 137, respectively, adds the acquired time to the acquired data, and supplies the resulting acquired data to the correction section 142.

The merge processing section 140 performs merge processing of HDR acceleration data and LDR acceleration data, and merge processing of HDR angular velocity data and LDR angular velocity data. The merge processing section 140 supplies the merged acceleration data and angular velocity data to the posture detection section 141 and the buffer 143.

The posture detection section 141 detects postures of the detection points on the basis of acceleration data, angular velocity data, and geomagnetic data. The posture of the detection point is represented by, for example, Euler angles. The posture detection section 141 supplies data indicating calculated results (hereinafter, referred to as posture data) to the buffer 143.

The correction section 142 corrects the strain data and the barometric pressure data, and supplies the corrected strain data and barometric pressure data to the buffer 143.

The buffer 143 temporarily accumulates posture data, acceleration data, angular velocity data, strain data, and barometric pressure data.

The packet generation section 144 generates a communication packet for transmission of data (hereinafter, referred to as motion detection data) including posture data, acceleration data, angular velocity data, strain data, and barometric pressure data accumulated in the buffer 143, and supplies the communication packet to the transmission control section 145.

The transmission control section 145 controls transmission of the communication packet by the transmission section 146.

The transmission section 146 transmits the communication packet to the motion recognizer 102 by wireless communication of a predetermined system.

It is to be noted that, although illustration is omitted, each section of the wearable sensor 101 is driven by, for example, a battery.

### <Configuration Example of Motion Recognizer 102>

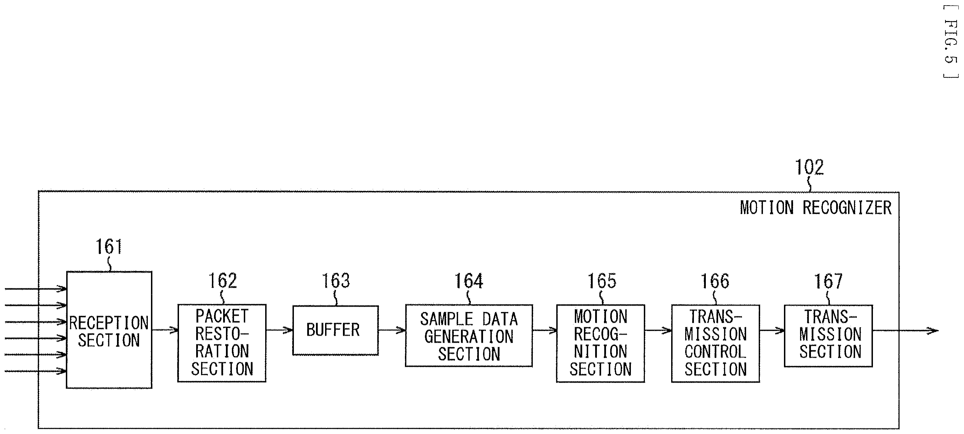

FIG. 5 illustrates a configuration example of the motion recognizer 102.

The motion recognizer 102 includes a reception section 161, a packet restoration section 162, a buffer 163, a sample data generation section 164, a motion recognition section 165, a transmission control section 166, and a transmission section 167.

The reception section 161 receives a communication packet from each wearable sensor 101 by wireless communication of a system corresponding to the transmission section 146 of each wearable sensor 101, and supplies the communication packet to the packet restoration section 162.

The packet restoration section 162 restores the communication packet of each wearable sensor 101, associates motion detection data included in the communication packet with a time stamp, and supplies the associated motion detection data to the buffer 163.

The buffer 163 temporarily accumulates the motion detection data and the time stamp extracted from the communication packet of each wearable sensor 101.

The sample data generation section 164 generates sample data for use in motion recognition processing using the motion detection data accumulated in the buffer 163, and supplies the sample data to the motion recognition section 165.

The motion recognition section 165 performs processing of motion recognition of the user on the basis of the sample data using a motion recognition model which is a model used for motion recognition of the user. The motion recognition section 165 supplies results of motion recognition of the user to the transmission control section 166.

The transmission control section 166 generates motion control data on the basis of results of the motion recognition of the user, and controls transmission of the motion control data by the transmission section 167. That is, the transmission control section 166 controls outputting of the motion control data from the motion recognizer 102.

The transmission section 167 transmits the motion control data to an apparatus in a subsequent stage by wireless communication or wired communication of a predetermined system.

### <Processing in Wearable Sensor 101>

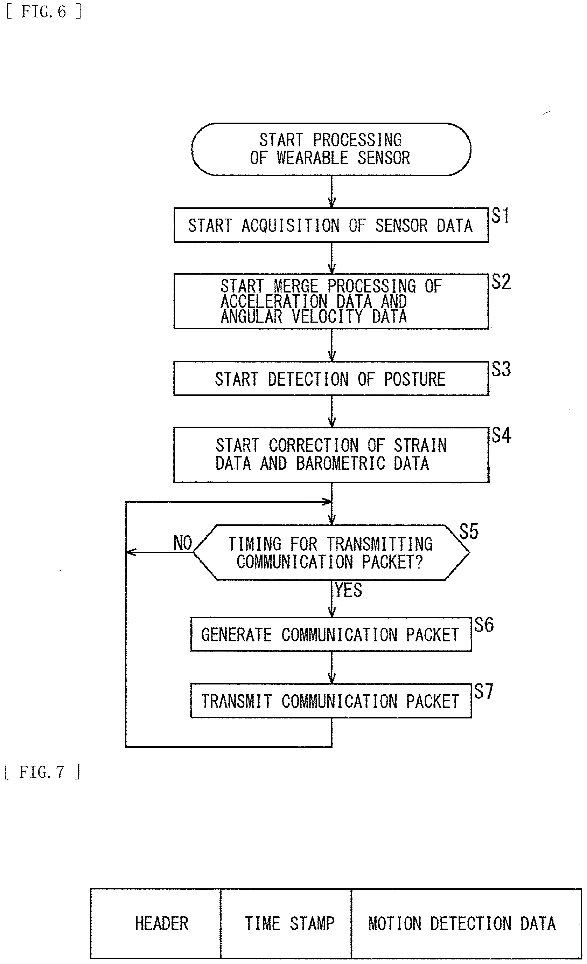

Next, with reference to a flowchart in FIG. 6, description is given of processing in the wearable sensor 101. This processing is started, for example, when the power of the wearable sensor 101 is turned on, and is finished when the power is turned off.

In step S1, the sensor data acquisition section 139 starts acquiring sensor data.

Specifically, the sensor data acquisition section 139 starts processing of acquiring HDR acceleration data, LDR acceleration data, HDR angular velocity data, and LDR angular velocity data from the HDR acceleration sensor 131, the LDR acceleration sensor 132, the HDR gyro sensor 133, and the LDR gyro sensor 134, respectively; adding the acquired time to the acquired data; and supplying the resulting acquired data to the merge processing section 140. In addition, the sensor data acquisition section 139 starts processing of acquiring geomagnetic data from the geomagnetic sensor 135, adding the acquired time to the acquired data, and supplying the resulting acquired data to the posture detection section 141. Further, the sensor data acquisition section 139 starts processing of acquiring strain data and barometric pressure data from the strain sensor 136 and the barometric sensor 137, respectively, adding the acquired time to the acquired data, and supplying the resulting acquired data to the correction section 142.

In step S2, the merge processing section 140 starts merging processing of the acceleration data and the angular velocity data.

Specifically, the merge processing section 140 starts processing of merging (synthesizing) the HDR acceleration data and the LDR acceleration data, and supplying the merged acceleration data to the buffer 143 for accumulation.

It is to be noted that, among the merged acceleration data, data based on the LDR acceleration data is used for data within the dynamic range of the LDR acceleration data, and data based on the HDR acceleration data is used for data outside the range. As a result, detection accuracy in the acceleration within the dynamic range of the LDR acceleration data is improved in the merged acceleration data.

In addition, the merge processing section 140 starts processing of merging (synthesizing) the HDR angular velocity data and the LDR angular velocity data, and supplying the merged angular velocity data to the buffer 143 for accumulation.

It is to be noted, among the merged angular velocity data, data based on the LDR angular velocity data is used for data within the dynamic range of the LDR angular velocity data, and data based on the HDR angular velocity data is used for data outside the range. As a result, in the merged angular velocity data, the detection accuracy in the angular velocity within the dynamic range of the LDR angular velocity data is improved.

In step S3, the posture detection section 141 starts detecting a posture. Specifically, the posture detection section 141 starts processing of detecting Euler angles around three axes, i.e., around the pitch axis, around the roll axis, and around the yaw axis, which indicate postures of the detection points, on the basis of the acceleration data, the angular velocity data, and the geomagnetic data. In addition, the posture detection section 141 starts processing of supplying posture data indicating the detection results to the buffer 143 for accumulation.

In step S4, the correction section 142 starts correcting the strain data and the barometric pressure data. For example, the correction section 142 starts processing of removing noises of the strain data and the barometric pressure data, converting data sizes, and the like. In addition, the correction section 142 starts processing of supplying the corrected strain data and barometric pressure data to the buffer 143 for accumulation.

In step S5, the packet generation section 144 determines whether or not it is a timing to transmit a communication packet. This determination processing is repeatedly executed until it is determined to be a timing to transmit the communication packet; in a case where it is determined to be a timing to transmit the communication packet, the processing proceeds to step S6.

It is to be noted that, for example, the communication packet is transmitted at predetermined intervals. For example, the communication packet is transmitted at a frequency of transmission of 128 Hz (i.e., 128 times per second). In this case, the sampling frequency of the motion detection data included in the communication packet is 128 Hz.

In step S6, the packet generation section 144 generates a communication packet. For example, the packet generation section 144 reads out the latest posture data, acceleration data, angular velocity data, strain data, and barometric pressure data from the buffer 143. In addition, the packet generation section 144 acquires time information indicating the current time from the time information acquisition section 138. Then, the packet generation section 144 generates a communication packet including the posture data, the acceleration data, the angular velocity data, the strain data, and the barometric pressure data read out from the buffer 143.

FIG. 7 illustrates an example of a format of a communication packet generated by the packet generation section 144. The communication packet includes a header, a time stamp, and motion detection data.

For example, time at which the sensor data is acquired is set in the time stamp. It is to be noted that the time at which the sensor data is acquired is set to, for example, the earliest of acquisition times of the respective sensor data used for the motion detection data in the communication packet. In addition, for example, in a case where acquisition timings of the respective data included in the motion detection data differs, the time stamp (time at which the sensor data is acquired) may be stored in the communication packet for each data. In this case, the format of the communication packet is, for example, a header, a time stamp 1, data 1 (e.g., posture data) included in the motion detection data, time stamp 2, data 2 (e.g., acceleration data) included in the motion detection data, and so on.

The motion detection data includes posture data, acceleration data, angular velocity data, strain data, and barometric pressure data read out from the buffer 143. It is to be noted that the motion detection data is encrypted by a predetermined method, for example.

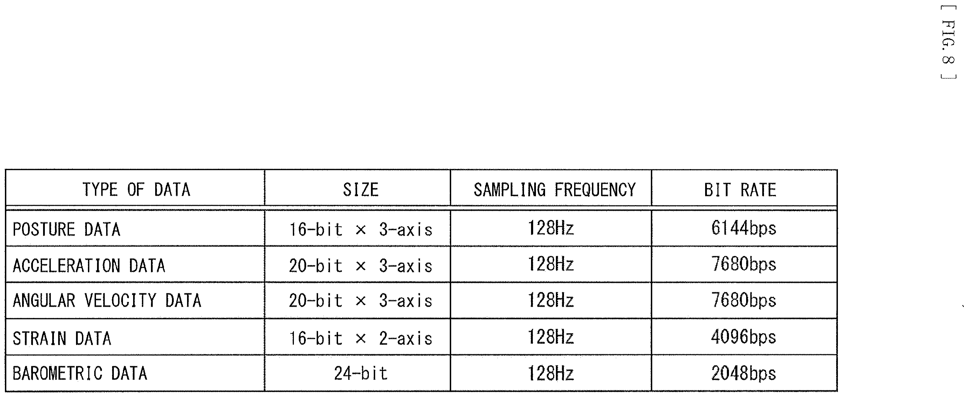

FIG. 8 illustrates an example of specifications of each data included in the motion detection data.

The size of the posture data is set to, for example, 16 bits×3 axes. In a case where the frequency with which the communication packet is transmitted is 128 Hz, the sampling frequency of the posture data is 128 Hz, and the bit rate thereof is 6,144 bps (bit per second).

The size of the acceleration data is, for example, 20 bits×3 axes. In a case where the frequency with which the communication packet is transmitted is 128 Hz, the sampling frequency of the acceleration data is 128 Hz, and the bit rate thereof is 7,680 bps.

The size of the angular velocity data is, for example, 20 bits×3 axes. In a case where the frequency with which the communication packet is transmitted is 128 Hz, the sampling frequency of the angular velocity data is 128 Hz, and the bit rate thereof is 7,680 bps.

The size of the strain data is, for example, 16 bits×2 axes. In a case where the frequency with which the communication packet is transmitted is 128 Hz, the sampling frequency of the strain data is 128 Hz, and the bit rate thereof is 4,096 bps.

The size of the barometric pressure data is, for example, 24 bits. In a case where the frequency with which the communication packet is transmitted is 128 Hz, the sampling frequency of the barometric pressure data is 128 Hz, and the bit rate thereof is 2,048 bps.

The packet generation section 144 supplies the communication packet to the transmission control section 145. Further, the packet generation section 144 deletes, from the buffer 143, each data transmitted by a communication packet and data older than each data.

In step S7, the transmission section 146 transmits a communication packet to the motion recognizer 102 under the control of the transmission control section 145.

Thereafter, the processing returns to step S5, and processing from step S5 onward is executed.

### <Processing in Motion Recognizer 102>

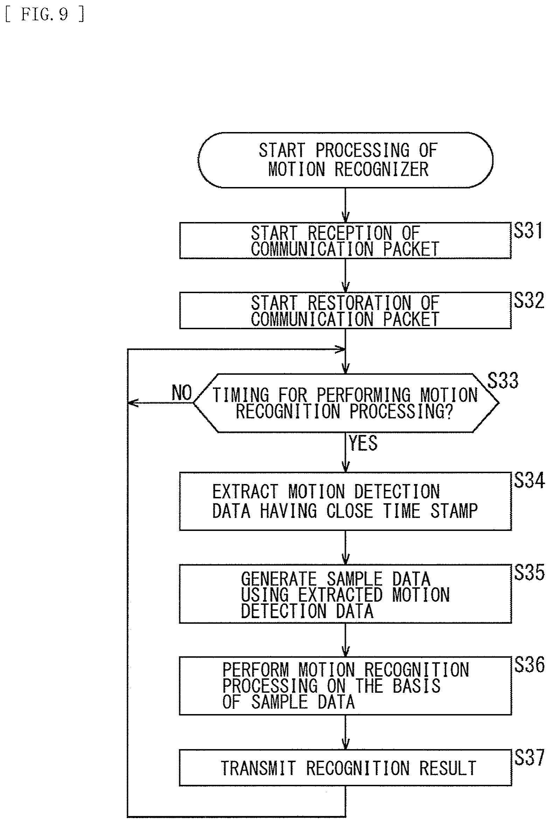

Next, with reference to a flowchart in FIG. 9, description is given of processing executed by the motion recognizer 102 in a manner corresponding to the processing of each wearable sensor 101 in FIG. 6. This processing is started, for example, when the power of the motion recognizer 102 is turned on, and is finished when the power is turned off.

In step S31, the reception section 161 starts receiving a communication packet. That is, the reception section 161 starts processing of receiving a communication packet transmitted from each wearable sensor 101 and supplying the received communication packet to the packet restoration section 162.

In step S32, the packet restoration section 162 starts restoring the communication packet. For example, after performing decoding, etc. of the motion detection data included in the communication packet received from each wearable sensor 101, the packet restoration section 162 starts processing of associating the motion detection data with the time stamp included in the communication packet, and supplying the associated motion detection data to the buffer 163 for accumulation.

In step S33, the sample data generation section 164 determines whether or not it is a timing to perform the motion recognition processing. This determination processing is repeatedly executed until it is determined to be a timing to perform the motion recognition processing; in a case where it is determined to be a timing to perform the motion recognition processing, the processing proceeds to step S34.

It is to be noted that, for example, the motion recognition processing is performed at predetermined intervals. For example, the motion recognition processing is executed with the same frequency as the frequency with which the communication packet is transmitted (e.g., 128 times per second).

In step S34, the sample data generation section 164 extracts motion detection data having a close time stamp.

For example, on the basis of the time stamp of each motion detection data accumulated in the buffer 163, the sample data generation section 164 detects a time zone in which the time stamp of the motion detection data of all the detection points falls within a range of predetermined time period (e.g., several milliseconds) while going back from the current time to the previous time. Then, the sample data generation section 164 extracts the motion detection data with the time stamp included in the time zone. As a result, pieces of motion detection data having a close time stamp are extracted one by one for the respective detection points. That is, pieces of the motion detection data corresponding to the movements detected at substantially the same timing at the respective detection points are extracted.

It is to be noted that, in a case where a time zone is not detected in which the time stamp of the motion detection data of all the detection points falls within a range of predetermined time period, for example, a time zone in which the number of the detection data is maximized may be detected to extract the motion detection data in that time zone. In this case, the motion detection data of some of the detection points is lost. Alternatively, the width of the time zone in which the motion detection data is extracted may be widened to allow the motion detection data of all the detection points to be extracted.

In step S35, the sample data generation section 164 generates sample data using the extracted motion detection data. Specifically, the sample data generation section 164 generates sample data including the motion detection data of the respective detection points extracted in the processing of step S34, and supplies the sample data to the motion recognition section 165. In addition, the sample data generation section 164 deletes, from the buffer 163, the extracted motion detection data and the motion detection data with the time stamp being older than that of the extracted motion detection data.

In step S36, the motion recognition section 165 performs the motion recognition processing on the basis of the sample data. For example, the motion recognition section 165 recognizes, using the motion recognition model, the motion of the user on the basis of the postures, accelerations, angular velocities, strains (expansion and contraction), and barometric pressures (heights) of the respective detection points of the user.

It is to be noted that the motion recognition model is, for example, a model that performs the motion recognition of a user on the basis of a motion relationship among the respective detection points of the user (e.g., interlocking property among the respective detection points, etc.). The motion recognition model is generated by, for example, preliminary learning processing. It is possible to adopt, as a learning method of the motion recognition model, any method such as a recursive neural network (RNN).

The motion recognition section 165 supplies the results of the motion recognition of the user to the transmission control section 166.

In step S37, the motion recognizer 102 transmits the recognition results. Specifically, the transmission control section 166 generates motion control data on the basis of the results of the motion recognition of the user, and supplies the motion control data to the transmission section 167.

It is to be noted that the motion control data includes, for example, at least one of the results of the motion recognition of the user or control information on processing corresponding to the recognized motion.

In addition, the motion control data may include motion detection data of respective detection points used for motion recognition. This makes it possible to detect details of the recognized motion on the basis of the motion detection data in an apparatus in a subsequent stage.

Then, the transmission section 167 transmits the motion control data to an apparatus in a subsequent stage, for example, a smartphone, a personal computer, various systems, or the like.

It is to be noted that the transmission of the motion control data may be performed by intermittent transmission. For example, the motion control data may be transmitted at a timing at which the results of the motion recognition change. This makes it possible to reduce the power consumption of the motion recognizer 102.

In addition, for example, a general-purpose protocol such as HID (Human Interface Device) or MIDI (Music Instrument Digital Interface) may be used as a transmission protocol of the motion control data. This makes it possible to use existing software or the like in an apparatus in a subsequent stage.

Thereafter, the processing returns to step S33, and the processing from step S33 onward is executed.

As described above, it is possible to recognize the motion of the user easily and accurately.

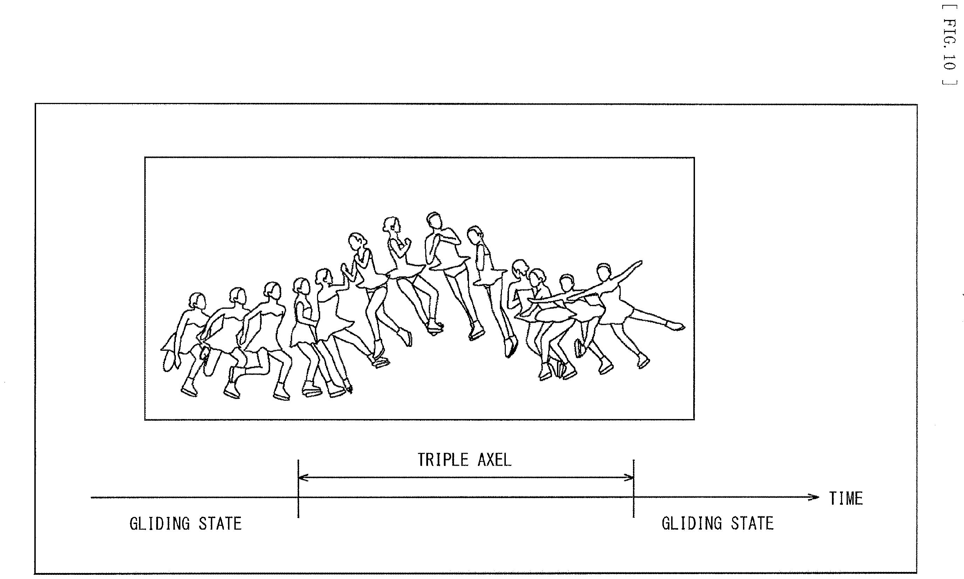

For example, as illustrated in FIG. 10, in a case where the user is performing figure skating, it is possible to recognize individual techniques such as triple axel and detect a period during which the technique is being performed.

Alternatively, for example, it is possible to recognize, easily and accurately, individual movements such as "standing", "crouching", "sitting", "rising", "lying", "walking", "running", "stopping", "jumping", "crawling", "ducking", "shaking", "throwing" and "kicking" that constitute an action of the user.

It is to be noted that the motion recognition system 100 recognizes types of motion of the user (e.g., information on meaning of the motion), but does not further perform recognition of a detailed movement of each motion.

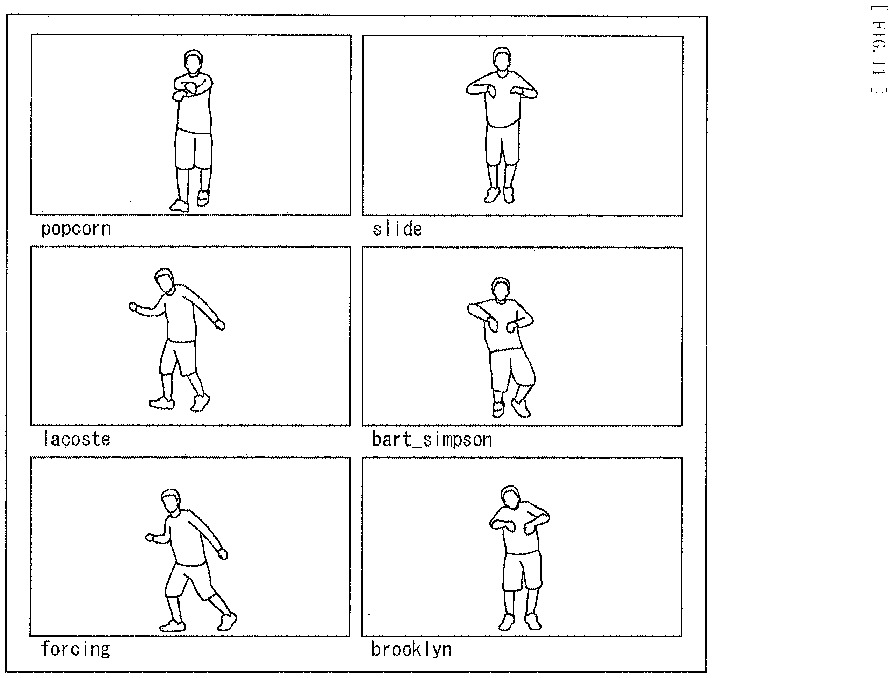

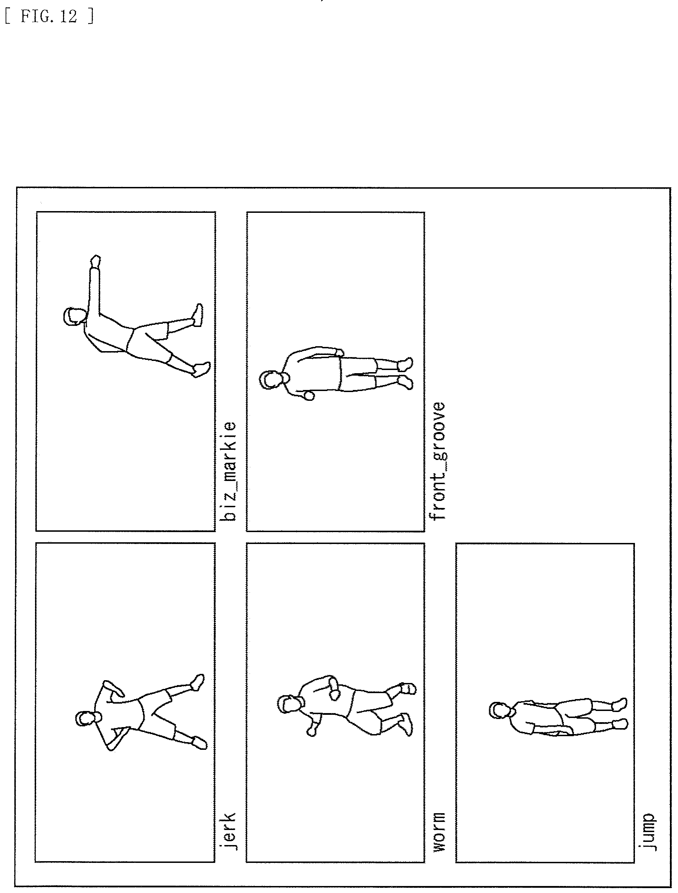

For example, in a case where the user is dancing, as illustrated in FIGs. 11 and 12, types of individual techniques of dances such as popcorn, slide, lacoste, bart simpson, forcing, brooklyn, jerk, biz markie, worm, front groove, and jump is recognized. On the other hand, differences in movements in individual techniques are not recognized. That is, even when the user performs the same dance technique by changing the movement or rhythm of limbs, the technique is recognized as the same dance technique, and no further difference is recognized.

It is to be noted that the number of the wearable sensors 101 attached may be reduced by thus limiting to types of motions of the user based on predetermined classification. As a result, it is possible to reduce the burden on the user as compared with an existing motion capture system used in CG (Computer Graphics) or the like. For example, even when the user wears the wearable sensor 101, it is possible for the user to perform substantially usual movement with little restriction on the movement.

In the existing motion capture system, information on the position and posture of the entire body of the user is outputted. Then, for example, a developer of an application program needs to independently develop an algorithm for motion recognition of the user on the basis of the information and to incorporate the algorithm into the application program.

Meanwhile, in the motion recognition system 100, the types of motions are recognized and outputted, and thus the burden on the developer of the application programs is reduced.

In addition, for example, as compared with a method of performing the motion recognition of a user on the basis of an image filmed by installing a camera, restriction on a place or an environment, etc. where motion recognition is performed is reduced.

For example, it is possible to perform motion recognition without paying attention to the installation position, the filming range, the filming condition, and the like of the camera. For example, it is possible to perform motion recognition while the user is freely acting without paying attention to the filming range or occlusion of the camera. For example, it is possible to recognize a motion of the user in a dark place, at sea (e.g., window surfing, etc.) or the like. Further, for example, it is possible for the user who is a recognition target to wear clothes or costumes of a desired color or design without paying attention to the relationship with the set of stages, the background, or the like. In addition, for example, the position at which the camera is installed is not limited by structure, rendering, or the like of the stage, and as a result a range in which the motion of the user is recognizable is not limited.

Further, by providing the motion recognition system 100 for each user, it is possible to individually recognize the motions of each user without being influenced by other users. For example, it is possible, in a dance performance in a group, to recognize the motion of an individual performer, or the motion of a specific performer.

In addition, the sampling frequencies of the respective sensors of the wearable sensor 101 are higher than the frame rate of a typical camera, thus making it possible to recognize higher-speed motions of the user. For example, it is possible to accurately recognize high-speed movements such as dance turns and steps.

It is to be noted that a camera may be provided in the motion recognition system 100 and the position of the user may be detected on the basis of an image filmed by the camera. Alternatively, at least one of the wearable sensors 101 may be provided with a device that is able to detect position information of the user, such as a GNSS (Global Navigation Satellite System) receiver to allow for detection of the position of the user. This makes it possible to detect the movement of the user in more detail on the basis of results of the motion recognition and detection results of the position information.

### <Configuration Example of Learning System >

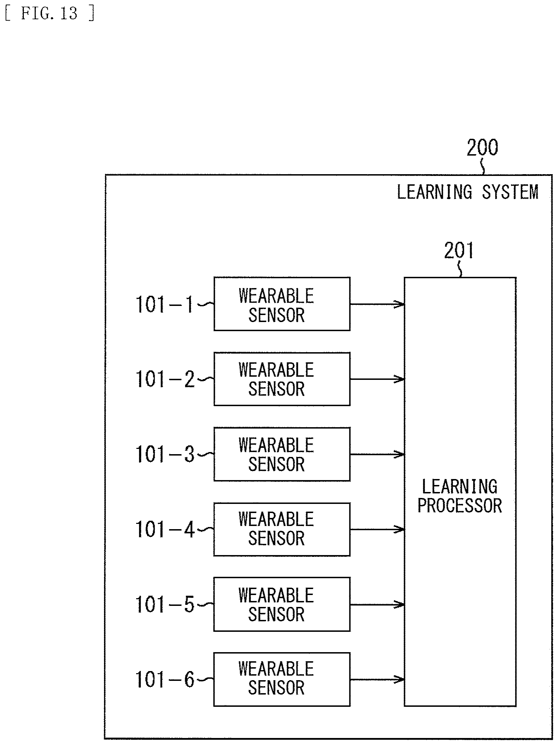

FIG. 13 illustrates a configuration example of a learning system 200 which is an embodiment of a learning system to which the present technology is applied. It is to be noted that, in the diagram, parts corresponding to those of the motion recognition system 100 in FIG. 1 are denoted by the same reference numerals and descriptions thereof are omitted as appropriate.

The learning system 200 is a system that performs learning processing of the motion recognition model to be applied to the motion recognition section 165 of the motion recognizer 102.

The learning system 200 includes the wearable sensor 101-1 to the wearable sensor 101-6 and a learning processor 201.

The learning processor 201 performs learning processing of the motion recognition model on the basis of motion detection data included in a communication packet transmitted from each wearable sensor 101.

### <Configuration Example of Information Processor>

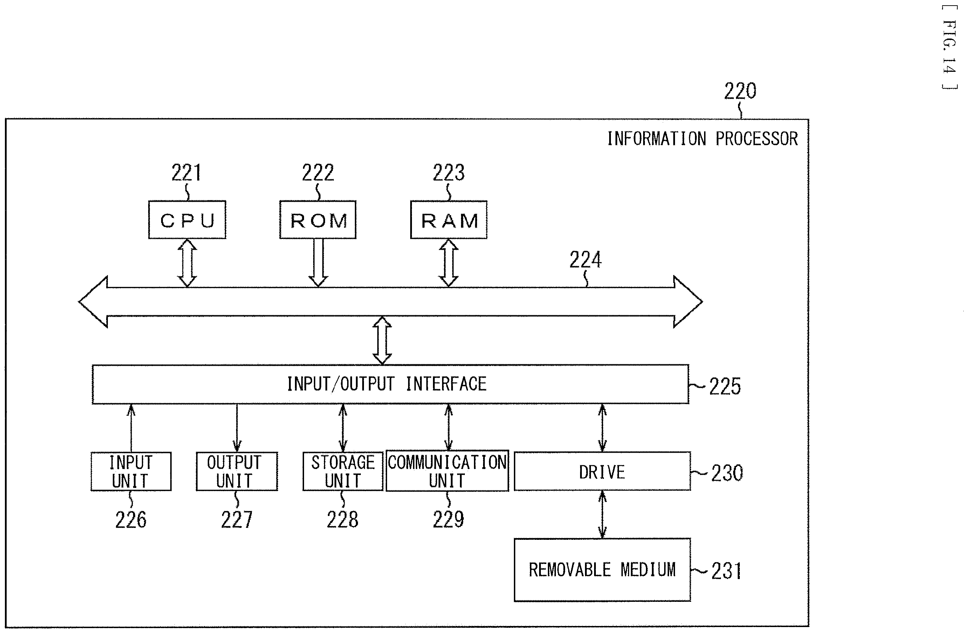

FIG. 14 illustrates a configuration example of an information processor 220 applicable to the learning processor 201 in FIG. 13.

The information processor 220 is configured by, for example, a personal computer, a smart phone, a tablet, or the like. The information processor 220 includes a CPU (Central Processing Unit) 221, a ROM (Read Only Memory) 222, a RAM (Random Access Memory) 223, a bus 224, an input/output interface 225, an input unit 226, an output unit 227, a storage unit 228, a communication unit 229, a drive 230, and a removable medium 231.

The CPU 221, the ROM 222 and the RAM 223 are coupled to one another by a bus 224. The input/output interface 225 is further coupled to the bus 224. The input unit 226, the output unit 227, the storage unit 228, the communication unit 229, and the drive 230 are coupled to the input/output interface 225.

The input unit 226 includes, for example, a keyboard, a mouse, a microphone, and the like.

The output unit 227 includes, for example, a display, a speaker, and the like.

The storage unit 228 includes, for example, a hard disk, a non-volatile memory, and the like.

The communication unit 229 includes, for example, a wireless or wired communication device, a network interface, and the like.

The drive 230 drives a removable medium 231 such as a magnetic disk, an optical disk, a magneto-optical disk, or a semiconductor memory.

In the information processor 220, the CPU 221 loads a program stored in, for example, the storage unit 228 into the RAM 243 via the input/output interface 225 and the bus 224, and executes the program to thereby perform various types of processing.

The programs executed by the information processor 220 (CPU 221) may be provided by being recorded in the removable medium 231 as a package medium or the like, for example. In addition, the program may also be provided via a wired or wireless transmission medium, such as a local area network, the Internet, or digital satellite broadcasting.

In the information processor 220, a program may be installed in the storage unit 228 via the input/output interface 225 by mounting the removable medium 231 in the drive 230. In addition, the program may also be received by the communication unit 229 via a wired or wireless transmission medium and installed in the storage unit 228. In addition, the program may be installed in advance in the ROM 202 or the storage unit 228.

It is to be noted that, in the following, descriptions of the bus 224 and the input/output interface 225 are omitted as appropriate. For example, in a case where the CPU 221 and the communication unit 229 transmit and receive data via the bus 224 and the input/output interface 225, description is simply given to the effect that the CPU 221 and the communication unit 229 transmit and receive data.

### <Configuration Example of Learning Processing Unit>

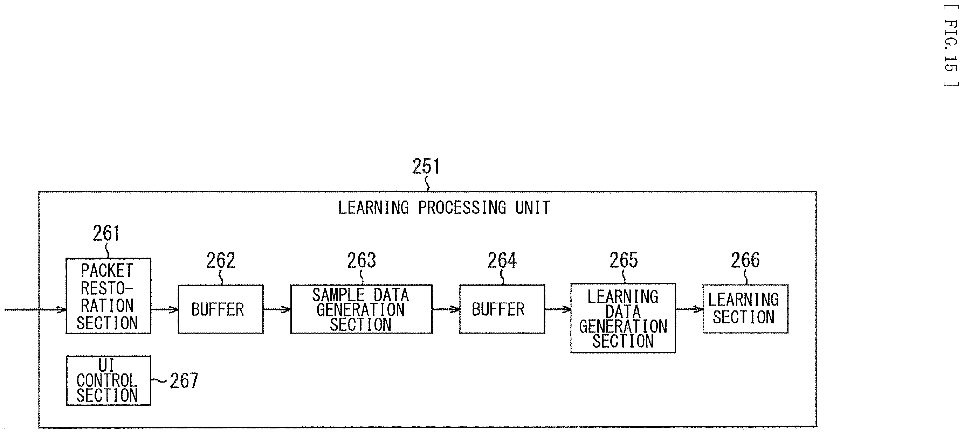

FIG. 15 illustrates a configuration example of a learning processing unit 251 achieved by the CPU 221 of the information processor 220 executing a predetermined control program.

The learning processing unit 251 includes a packet restoration section 261, a buffer 262, a sample data generation section 263, a buffer 264, a learning data generation section 265, a learning section 266, and a UI (user interface) control section 267.

Similarly to the packet restoration section 162 in FIG. 5, the packet restoration section 261 restores a communication packet of each wearable sensor 101, associates motion detection data included in a communication packet with a time stamp, and supplies the associated motion detection data to the buffer 262.

The buffer 262 temporarily accumulates the motion detection data extracted from the communication packet of each wearable sensor 101.

Similarly to the sample data generation section 164 in FIG. 5, the sample data generation section 263 generates sample data using the motion detection data accumulated in the buffer 262, and supplies the sample data to the buffer 264.

The buffer 264 temporarily accumulates the sample data.

The learning data generation section 265 generates learning data by adding a label to the sample data accumulated in the buffer 264, and supplies the learning data to the learning section 266. The label indicates, for example, types of motions of the user at the time when the motion detection data included in the sample data is detected.

The learning section 266 performs learning processing of a motion recognition model on the basis of the learning data. It is to be noted that any learning method may be adopted for the learning processing of the motion recognition model.

The UI control section 267 controls the output unit 227 on the basis of user input or the like inputted via the input unit 226, and controls the user interface at the time of performing the learning processing of the motion recognition mode.

### <Motion Registration Processing>

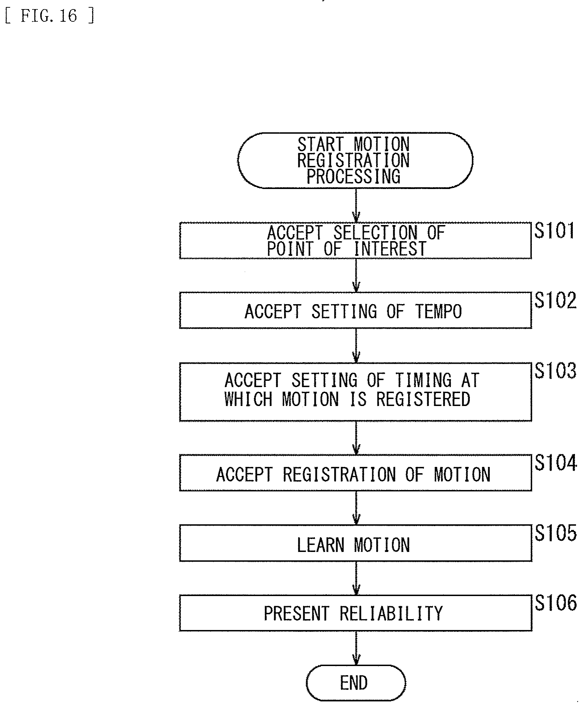

Next, with reference to a flowchart in FIG. 16, description is given of motion registration processing as an example of learning processing executed by the learning system 200. The motion registration processing is processing of registering a motion to be newly recognized in the motion recognition model. Hereinafter, description is given, as an example, of a case where a new dance technique is registered as a recognition target.

In step S101, the learning processing unit 251 receives selection of a point of interest.

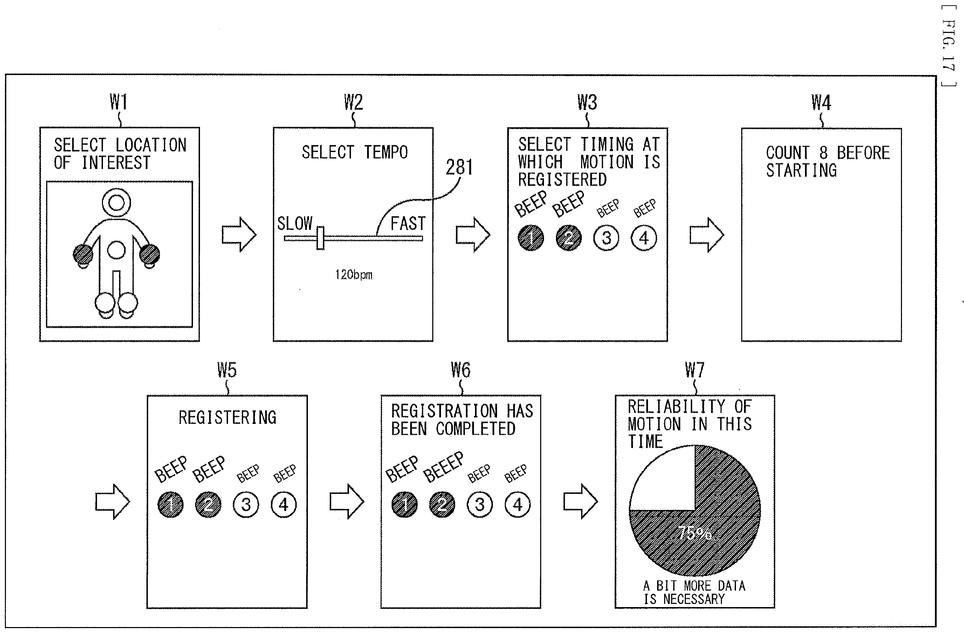

For example, the output unit 227 displays a window W1 in FIG. 17 under the control of the UI control section 267. In the window W1, locations (detection points) where the user wears the wearable sensor 101 are indicated by circles. Then, the user selects a point of interest from among the detection points via the input unit 226.

Here, the point of interest refers to a detection point (location) of particular interest in a motion to be registered. For example, a detection point in a location that particularly remarkably represents a feature of the motion to be registered is selected as the point of interest. Then, for example, in the learning processing, the movement of the point of interest is emphasized more than the movements of other detection points.

For example, in a case where a dance technique in which both hands are swung greatly is registered as a new motion, the movements of both hands are important, whereas the movements of other locations are not very important. Therefore, in this case, for example, as illustrated in the window W1 in FIG. 17, both wrists are selected as the point of interests.

The UI control section 267 acquires selection results of the points of interest from the input unit 226, and supplies the selection results to the learning section 266.

It is to be noted that points of interest necessarily need not be selected.

In step S102, the learning processing unit 251 accepts setting of a tempo.

For example, the output unit 227 displays a window W2 in FIG. 17 under the control of the UI control section 267. The window W2 displays a slider 281 for setting of the tempo of a clicking sound upon registering motions. For example, the user sets the tempo upon registering the motions by moving the slider 281 left and right via the input unit 226. In this example, the tempo is set to 120 bpm (beats per minute).

The UI control section 267 acquires setting results of the tempo from the input unit 226.

In step S103, the learning processing unit 251 receives setting of a timing for registering motions.

For example, the output unit 227 displays a window W3 in FIG. 17 under the control of the UI control section 267. The window W3 displays circled numbers 1 to 4 indicating four-beat clicking sounds. It is to be noted that, in the window W3 and windows W5 and W6 to be described later, characters such as "beep" over the circled number indicate clicking sounds to be outputted in respective beats, and are not actually displayed.

For example, the user sets a timing for registration of motions via the input unit 226. In this example, an example is illustrated in which the motion is set to be registered between clicking sounds of the first beat and the second beat. That is, a period from the output of the clicking sound of the first beat to the output of the clicking sound of the second beat is set as a registration period of the motion. In this case, for example, a sound volume of the clicking sounds of the first beat and the second beat becomes large, and a sound volume of the clicking sounds of the third beat and the fourth beat becomes small.

The UI control section 267 acquires setting results of the tempo from the input unit 226.

In step S104, the motion recognition system 100 accepts registration of motions.

For example, the output unit 227 displays a window W4 in FIG. 17 under the control of the UI control section 267, and presents a timing at which the registration of the motions is started. Then, under the control of the UI control section 267, the output unit 227 displays a window W5 in FIG. 17 at the period of time when the registration of the motions is started, and starts outputting the clicking sound at the set tempo. As described above, the sound volume of the clicking sound becomes large in the first beat and the second beat, and sound volume of the clicking sound becomes small in the third beat and the fourth beat.

It is to be noted that, for example, some of the wearable sensors 101 may have a built-in speaker to output a clicking sound from the speaker.

For example, the user performs a motion to be registered during a motion registration period between clicking sounds of the first beat and the second beat while wearing the wearable sensor 101. For example, the user starts the motion when the clicking sound of the first beat is outputted, and finishes the motion when the clicking sound of the second beat is outputted. This enables the user to reliably input motions into the learning system 200. In addition, this enables the user to register motions while having fun in a game feeling. It is to be noted that the clicking sound may be outputted in accordance with a desired music piece of the user to allow the user to register motions while having more fun.

Each wearable sensor 101 starts processing of transmitting a communication packet including motion detection data indicating the movement of each of the detection points at a predetermined cycle by the processing described above with reference to FIG. 6.

The communication unit 229 of the information processor 220 (learning processor 201) starts processing of receiving a communication packet from each wearable sensor 101 and providing the communication packet to the packet restoration section 261.

Through the processing similar to that of step S32 in FIG. 9, the packet restoration section 261 starts processing of restoring the communication packet of each wearable sensor 101, associating the motion detection data included in the communication packet with the time stamp, and supplying the associated motion detection data to the buffer 262 for accumulation.

Through the processing similar to that in steps S34 and S35 in FIG. 9, the sample data generation section 263 starts processing of generating sample data using the motion detection data accumulated in the buffer 262, and supplying the sample data to the buffer 264 for accumulation. It is to be noted that, at this time, for example, sample data is generated using only motion detection data detected between clicking sounds of the first beat and the second beat.

In step S105, the learning processing unit 251 learns the motions. Specifically, the learning data generation section 265 starts processing of generating learning data by adding a label indicating types of motions to be newly registered in the sample data accumulated in the buffer 264 and supplying the learning data to the learning section 206.

The learning section 206 starts learning processing for registration of a new motion in the motion recognition model on the basis of the acquired learning data. At this time, in a case where points of interest are selected, for example, the learning section 206 performs the learning processing by giving larger weight to the motion detection data of the points of interest than weight to the motion detection data of other detection points. That is, the learning section 206 performs learning processing by giving weights to the respective detection points.

Then, when the registration of a new motion is completed, the output unit 227 displays a window W6 in FIG. 17 under the control of the UI control section 267, notifies the completion of the registration of the motion, and outputs a clicking sound of "beeep" which differs from the clicking sound at the time of registration.

In step S106, the information processor 220 (learning processor 201) presents reliability. Specifically, the learning section 206 calculates the reliability of the currently registered motion by a predetermined method. The reliability of the motion is calculated on the basis of, for example, an amount of learning data used for registration of the motion, variance of sample data included in the learning data, probability of erroneously determining to be another motion, and the like. The higher the reliability of the motion is, the higher the accuracy in the motion recognition becomes; the higher the reliability of the motion is, the lower the recognition accuracy in the motion becomes.

In addition, the output unit 227 displays a window W7 in FIG. 17 under the control of the UI control section 267, and presents reliability of the currently registered motions. Further, in a case where the reliability of the motion is not sufficient, for example, a message is displayed to the effect that a little more learning data is necessary. This enables the user, for example, to make determination as to whether or not more learning is necessary, or whether or not to change the motion in order to clarify the difference from other motions.

Thereafter, the motion registration processing is finished.

In this manner, it is possible for the user to register a new motion easily while having fun in accordance with an easy-to-understand user interface. Further, by setting the point of interests, registration of motions is made efficient, thus, for example, making it possible to register a new motion with high recognition accuracy with fewer learning data.

### <Method for Improving Recognition Accuracy of Motion Recognition Model>

Next, description is given of an example of a method for improving recognition accuracy in the motion recognition model. Specifically, description is given of an example of a case of improving the recognition accuracy in a motion recognition model for recognition of each individual technique of a dance as a motion.

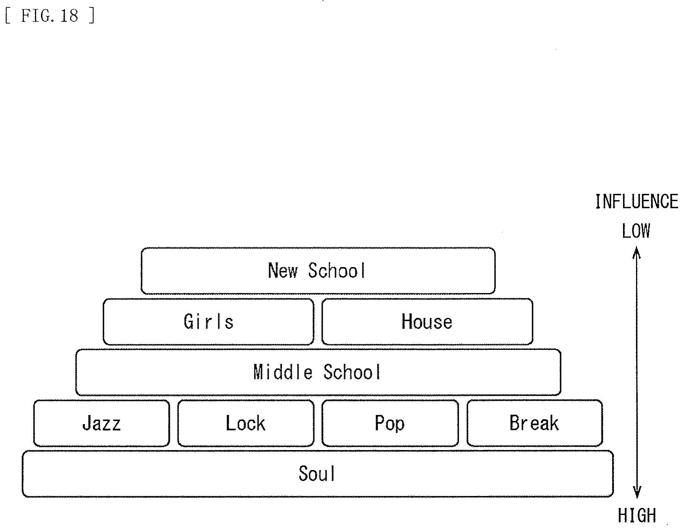

FIG. 18 illustrates an example in which genre of dances is organized. As illustrated in this diagram, the genre of dances is represented by a hierarchical structure. That is, the lower the genre in the diagram is, the older the occurrence time is; the higher the genre is, the newer the occurrence time is. Then, a new genre is created on the basis of the old genre.

For example, combining or refining the techniques of the old genre creates some of the techniques of a new genre. Therefore, a feature of the dance technique of the old genre is included in a portion of the technique of the new genre. Therefore, the older the genre is, the greater the influence on other genres becomes; the newer the genre is, the smaller the influence on other genres becomes.

For example, Jazz, Rock, Pop, and Break are genres each created on the basis of Soul. Middle School is a genre created on the basis of Jazz, Rock, Pop, and Break. Girls and House are genres each created on the basis of Middle School. New School is a genre created on the basis of Girls and House.

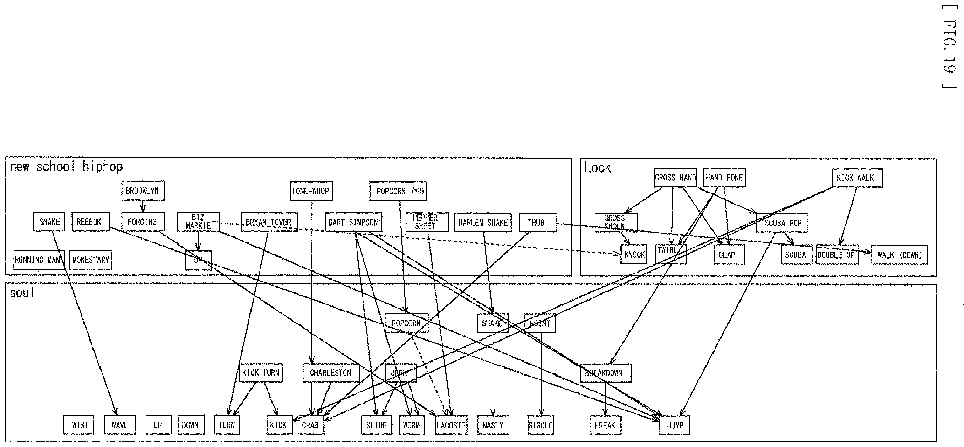

In addition, a dance technique of the new genre is created on the basis of the dance technique of the old genre, and thus dance techniques may also be represented by a hierarchical structure as illustrated in FIG. 19. It is to be noted that, in FIG. 19, relationships among dance techniques are indicated by arrows. That is, among the techniques connected by the arrows, the original technique of the arrow is influenced by the technique of the point of the arrow. For example, jerk is influenced by slide and worm. More specifically, a technique that combines slide and worm is jerk.

In this manner, by hierarchizing the dance techniques and arranging the master-slave relationships, it is possible to clarify the relationship between the features of respective dance techniques and to clarify the difference between the features of the respective techniques. Then, by learning the motion recognition model for recognition of the dance technique using the arranged results, it is possible to improve the recognition accuracy in the dance technique.

### <Method for Improving Real-Time Property of Motion Recognition>

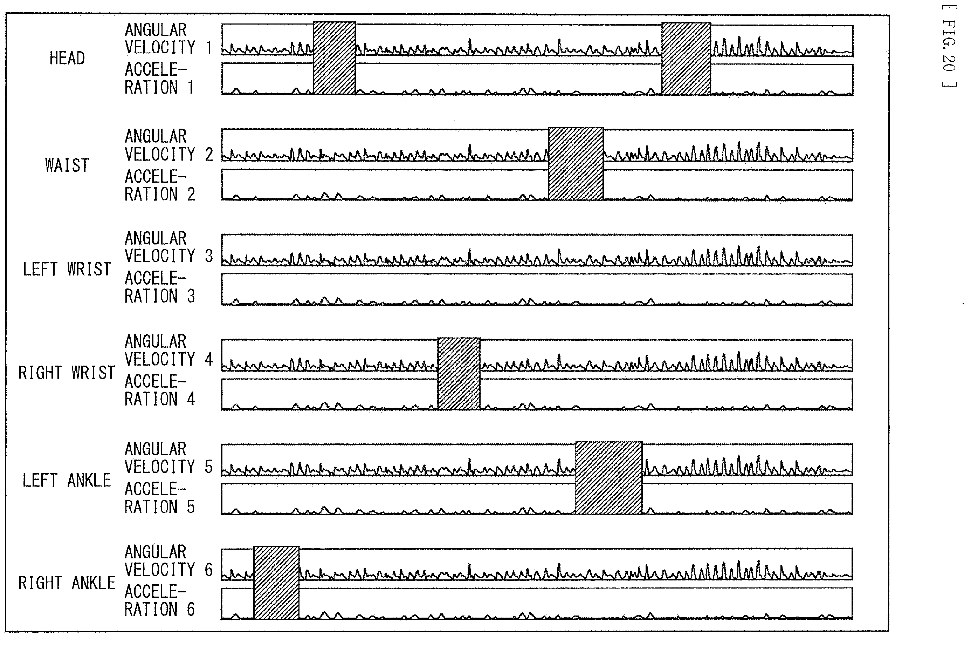

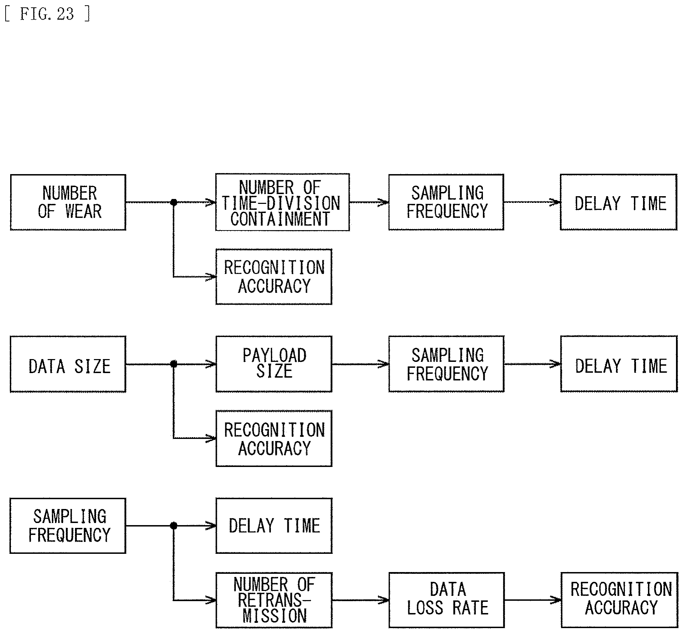

Next, with reference to FIGs. 20 and 23, description is given of a method for improving a real-time property of motion recognition in the motion recognition system 100.

For example, depending on cases of using the results of the motion recognition, a real-time motion property in the motion recognition is sometimes required. In other words, there are cases where it is required to shorten the time required from the time when a user performs a certain motion to the time when the motion recognition is performed (hereinafter, referred to as a delay time) as much as possible. Examples of the above-mentioned cases include a case where stage rendering is performed to follow the movement of a performer, a case where recognition results of the movement of a player is immediately fed back in a training of sports, etc., and a case where commands are inputted by the movements of a player in a game in which the real-time property is emphasized.

Meanwhile, in the existing wireless communication, it is generally important to reliably transmit and receive data, and specifications for retransmitting data is incorporated in a case where a communication error occurs. However, for example, in a case where communication errors occur between the wearable sensor 101 and the motion recognizer 102, and retransmission of the communication packet is repeated, delay time becomes longer by that amount. In addition, for example, when trying to perform the motion recognition processing after the communication packets of all the detection points are arranged, time period (delay time) from the time when the user performs the motion to the time when the motion recognition is performed becomes longer, and the real-time property is not secured.

Meanwhile, for example, when the number of retransmissions of the communication packet is limited in order to emphasize the real-time property, it is highly probable that a situation occurs where a communication packet (motion detection data) from some of the wearable sensors 101 are lost. That is, it is highly probable that the motion recognizer 102 is not notified of the movement of some of the detection points.

However, the motion recognition section 165 of the motion recognizer 102 performs the motion recognition of the user on the basis of the motion of the plurality of detection points of the user. Therefore, even when the communication packets of some detection points are lost, and the movements of the detection points are not notified, it is often the case that it is possible to accurately recognize the motion of the user on the basis of the movements of other detection points.

Therefore, in order to secure the real-time property of the motion recognition without decreasing the recognition accuracy, for example, it is considered that the learning system 200 learns the motion recognition model on the assumption that the communication packet (motion detection data) is lost, and the motion recognition system 100 limits the number of retransmissions of the communication packet.

Specifically, for example, in the learning section 266 of the learning system 200, it is conceivable to perform learning processing of the motion recognition model using learning data having partially lost motion detection data of respective detection points, in accordance with a method such as dropout.

For example, FIG. 20 illustrates some of examples of such learning data. Specifically, FIG. 20 illustrates an example of waveforms of angular velocity data and acceleration data among the motion detection data of the respective detection points included in the learning data. In each graph, the horizontal axis represents time, and the vertical axis represents angular velocity or acceleration. Further, in this diagram, the waveforms of the angular velocity data and the waveforms of the acceleration data at the respective detection points are substantially the same; however actually, the waveforms are different between the respective detection points.

It is to be noted that the hatched parts of the respective graphs in the diagram indicate parts in which motion detection data is lost. As described above, by performing the learning processing using the learning data in which the motion detection data of each detection point is partially lost, a motion recognition model that is robust against the loss of the motion detection data is constructed.

It is to be noted that, for example, it is desirable to measure a communication status between each wearable sensor 101 and the motion recognizer 102 and to use learning data in which the motion detection data is lost to be closer to the actual status on the basis of analysis results of tendency of occurrence of communication error.

Then, in the motion recognition system 100, the number of retransmissions of the communication packet between each wearable sensor 101 and the motion recognizer 102 is limited by using the motion recognition model that is robust against the loss of motion detection data.

For example, in a case where the frequency with which the communication packet is transmitted is 128 Hz, transmission interval of the communication packet is about 7.8 milliseconds. In this case, for example, it is conceivable to limit the number of retransmissions to allow the transmission of the communication packet to be completed within about 7.8 milliseconds.

However, the number of retransmissions of the communication packet and the loss rate of motion detection data are in a trade-off relationship. In addition, the loss rate of motion detection data and the motion recognition accuracy are also in a trade-off relationship.

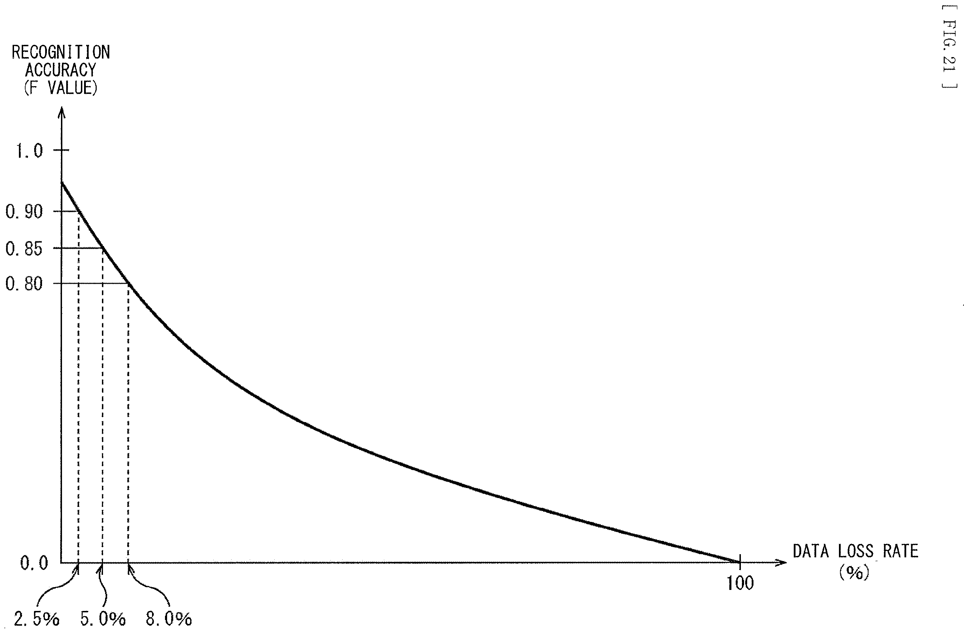

Specifically, FIG. 21 is a graph illustrating a relationship between the loss rate of motion detection data and the motion recognition accuracy (F value). The horizontal axis represents the data loss rate (unit: %), and the vertical axis represents the recognition accuracy.

As illustrated in this graph, the lower the loss rate of the motion detection data is, the higher the accuracy in the motion recognition becomes; the higher the loss rate of the motion detection data is, the lower the accuracy in the motion recognition becomes.

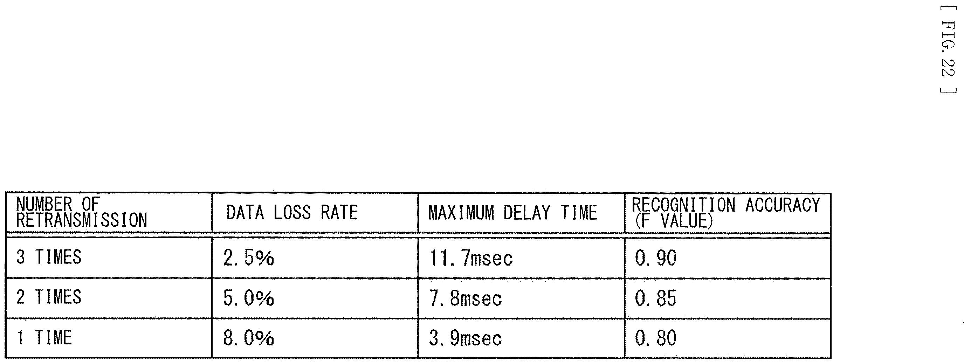

In addition, FIG. 22 illustrates an example of a relationship among the upper limit value of the number of retransmissions of the communication packet, the loss rate of the motion detection data, the maximum delay time of the communication packet, and the recognition accuracy (F value) in the motion.

For example, in a case where the number of retransmissions of the communication packet is limited to three, the loss rate of the motion detection data is 2.5%, the maximum delay time is 11.7 milliseconds, and the motion recognition accuracy is 0.90.

On the other hand, in a case where the number of retransmissions of the communication packet is limited to two, the loss rate of the motion data increases to 5.0%, and the motion recognition accuracy decreases to 0.85. Meanwhile, the maximum delay time is reduced to 7.8 milliseconds.

Further, in a case where the number of retransmissions of the communication packet is limited to one, the loss rate of the motion data increases to 8.0%, and the motion recognition accuracy decreases to 0.80. Meanwhile, the maximum delay time is reduced to 3.9 milliseconds.

Therefore, for example, it is desirable to be able to change the number of retransmissions of the communication packet of each wearable sensor 101, in accordance with the case of utilizing the results of the motion recognition.

For example, in a case where the real-time property of motion recognition is emphasized, the number of retransmissions of the communication packet is limited to one. This makes it possible to achieve an application that follows the motion of the user quickly, although the motion recognition accuracy is somewhat lowered.

Meanwhile, for example, in a case where the real-time property of the motion recognition is not so emphasized, the number of retransmissions of the motion is increased to three. This makes it possible to improve the motion recognition accuracy of the user, and thus to achieve an application that more appropriately follows the motion of the user.

It is to be noted that, in addition to the number of retransmissions, examples of factors contributing to the real-time property of the motion recognition include the number of the wearable sensors 101 attached, the size of motion detection data, and the sampling frequency of motion detection data (the frequency with which the communication packet is transmitted), as illustrated in FIG. 23.

For example, when the number of wearable sensor 101 attached is reduced, the number of the time-division containment is reduced, thus making it possible to increase the sampling frequency of the motion detection data (the frequency with which the communication packet is transmitted) without changing the number of retransmissions of the communication packet. As a result, the cycle of the motion recognition processing is shortened and the delay time is reduced, thus improving the real-time property of the motion recognition. On the other hand, when the number of the wearable sensors 101 attached is reduced, the number of detection points is reduced, thus lowering the motion recognition accuracy. Conversely, when the number of the wearable sensors 101 attached is increased, the real-time property of the motion recognition is lowered, but the motion recognition accuracy is improved.

Further, for example, when the size of the motion detection data is reduced, payload size of the communication packet is reduced, thus making it possible to increase the sampling frequency of the motion detection data (frequency with which the communication packet is transmitted) without changing the number of retransmissions of the communication packet. As a result, the cycle of the motion recognition processing is shortened and the delay time is reduced, thus making it possible to improve the real-time property of the motion recognition. On the other hand, when the size of the motion detection data is reduced, the size of each data included in the motion detection data is reduced, and the resolution of the acceleration, the angular velocity, and the like is lowered. As a result, the motion recognition accuracy is lowered. Conversely, when the size of the motion detection data is increased, the real-time property of the motion recognition decreases, but the motion recognition accuracy is improved.

Further, when the sampling frequency of the motion detection data (frequency with which the communication packet is transmitted) is increased, the cycle of the motion recognition processing becomes shorter, and the delay time decreases, thus improving the real-time property of the motion recognition. On the other hand, due to the shorter transmission cycle of the communication packet, it is necessary to reduce the number of retransmissions of the communication packet in a case where the delay time is tried to be contained within the transmission cycle of the communication packet. As a result, the data loss rate increases, and the motion recognition accuracy decreases. Conversely, when the sampling frequency of the motion detection data is lowered, the real-time property of the motion recognition is lowered, but the motion recognition accuracy is improved.

Accordingly, it is desirable to be able to adjust one or more of the number of retransmissions of the communication packet, the number of the wearable sensors 101 attached, the size of the motion detection data, and the sampling frequency of the motion detection data, on the basis of which of the real-time property of the motion recognition and the motion recognition accuracy is emphasized. This enables the motion recognition system 100 to appropriately cope with both an application requiring the real-time property and an application requiring the recognition accuracy.

### <<2. Second Embodiment>>

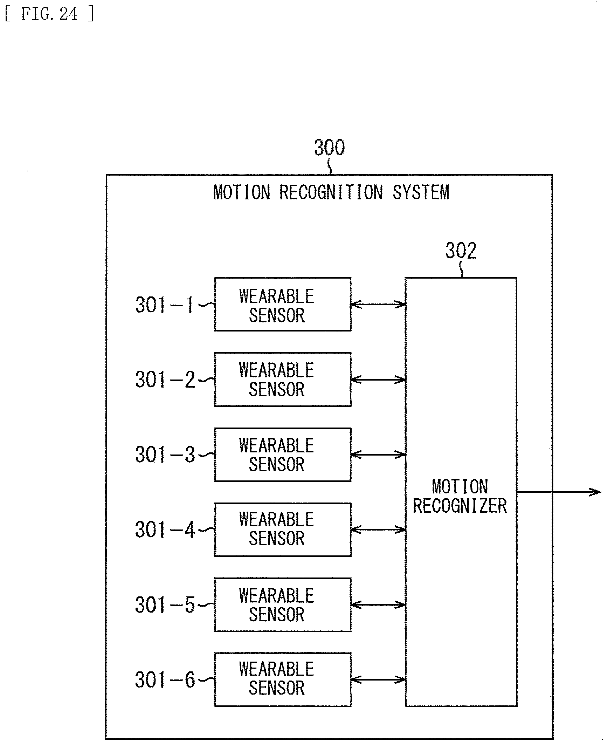

Next, with reference to FIGs. 24 to 30, description is given of a second embodiment of the present technology.

As described above, in the motion recognition system 100, the motion recognition is performed on the basis of the motion relationship between the detection points of the user. Accordingly, in order to improve the motion recognition accuracy, it is important to synchronize timings of detecting the movements of the respective detection points as much as possible. Alternatively, it is important to perform the motion recognition processing using the motion detection data detected at the respective detection points at the same timing as much as possible.

On the other hand, in the motion recognition system 100, the motion recognition processing is performed using the motion detection data detected at the respective detection points at the same time as much as possible on the basis of the time stamp of each communication packet.

However, in this case, when out-of-sync occurs to time of the time information acquisition section 138 of each wearable sensor 101, the difference in the detection timings of the motion detection data between the respective detection points becomes large. As a result, the motion recognition accuracy is lowered.

Accordingly, synchronization processing is required for the time between the time information acquisition sections 138 of the respective wearable sensors 101 by using some method. However, the synchronization processing at this time needs to be performed each time the power of each wearable sensor 101 is turned on, for example, thus causing high processing load. Further, more complicated processing is required to cope with the out-of-sync of the time during use of the motion recognition system 100.

Meanwhile, the detection timings of the motion detection data of the respective detection points used in the motion recognition processing necessarily need not be strictly simultaneous; it is often the case that some degree of out-of-sync (e.g., in units of several milliseconds or within one sample) is allowed.

Therefore, in the second embodiment, the motion recognizer controls the timing at which each wearable sensor detects the motion detection data, thereby performing the motion recognition processing at each detection point using the motion detection data detected at the same timing as much as possible.

### <Second Embodiment of Motion Recognition System>

FIG. 24 illustrates a configuration example of a motion recognition system 300 which is a second embodiment of the motion recognition system to which the present technology is applied.

The motion recognition system 300 includes a wearable sensor 301-1 to a wearable sensor 301-6 and a motion recognizer 302.

It is to be noted that, in the following, in a case where the wearable sensor 301-1 to the wearable sensor 301-6 need not be individually distinguished, they are simply referred to as a wearable sensor 301.

Similarly to each wearable sensor 101 in FIG. 1, each wearable sensor 301 is attached to a different detection point of the user. Each wearable sensor 301 detects the movement of each detection point under the control of the motion recognizer 302, and transmits a communication packet including detection results to the motion recognizer 302.

The motion recognizer 302 performs processing of motion recognition of the user on the basis of motion detection data of the detection points included in the communication packet received from each wearable sensor 301. The motion recognizer 302 transmits the motion control data based on results of the motion recognition of the user to an apparatus in a subsequent stage.

### <Configuration Example of Wearable Sensor 301>

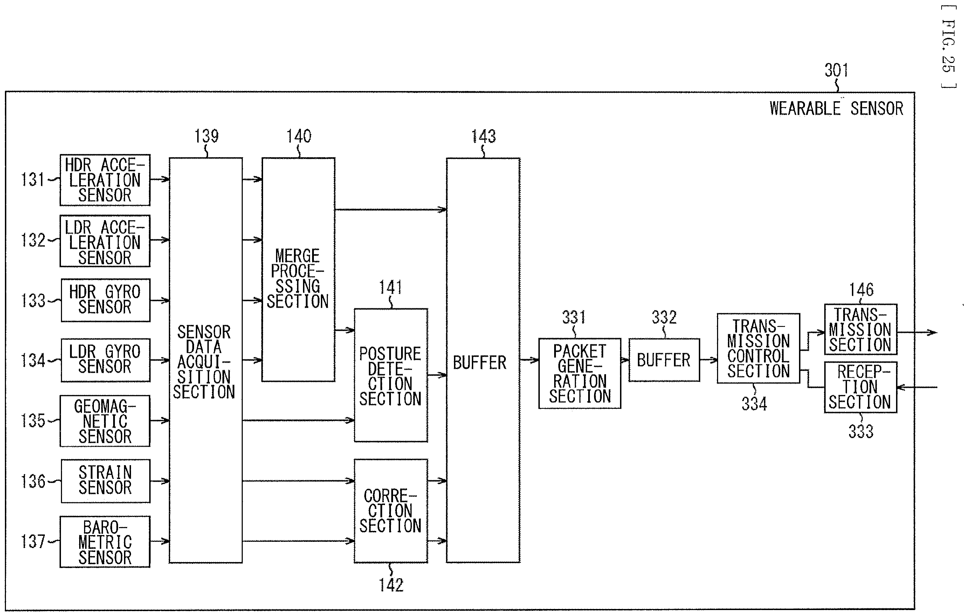

FIG. 25 illustrates a configuration example of the wearable sensor 301 in FIG. 24. It is to be noted that, in the diagram, parts corresponding to those of the wearable sensor 101 in FIG. 3 are denoted by the same reference numerals, and descriptions thereof are omitted as appropriate.

The wearable sensor 301 differs from the wearable sensor 101 in that a packet generation section 331 and a transmission control section 334 are provided instead of the packet generation section 144 and the transmission control section 145, that a buffer 332 and a reception section 333 are added, and that the time information acquisition section 138 is deleted.

The packet generation section 331 generates a communication packet for transmission of the motion detection data including posture data, acceleration data, angular velocity data, strain data, and barometric pressure data accumulated in the buffer 143, and supplies the communication packet to the buffer 332.

The buffer 332 temporarily accumulates communication packets.

The reception section 333 receives a transmission request signal requesting transmission of a communication packet from the motion recognizer 302 by wireless communication of the same system as the transmission section 146, and supplies the transmission request signal to the transmission control section 334.

The transmission control section 334 controls the transmission of the communication packet by the transmission section 146.

### <Configuration Example of Motion Recognizer 302>

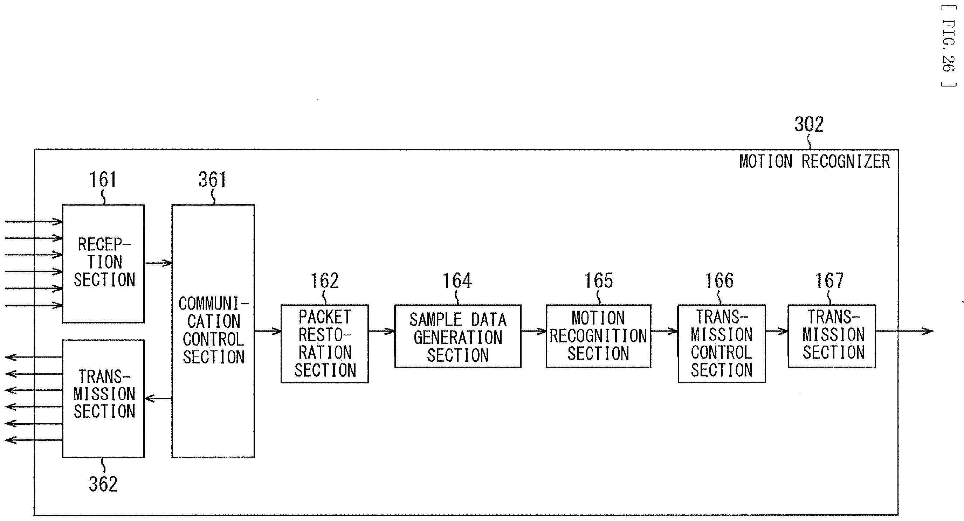

FIG. 26 illustrates a configuration example of the motion recognizer 302 in FIG. 24. It is to be noted that, in the diagram, parts corresponding to those of the motion recognizer 102 in FIG. 5 are denoted by the same reference numerals, and descriptions thereof are omitted as appropriate.

The motion recognizer 302 differs from the motion recognizer 102 in that a communication control section 361 and a transmission section 362 are added and the buffer 163 is deleted.

The communication control section 361 generates a transmission request signal for requesting each wearable sensor 301 to transmit a communication packet, and supplies the transmission request signal to the transmission section 362. Further, the communication control section 361 supplies the communication packet from each wearable sensor 301 supplied from the reception section 161 to the packet restoration section 162.

The transmission section 362 transmits the transmission request signal to each wearable sensor 301 by wireless communication of a system corresponding to the reception section 333 of each wearable sensor 301.

### <Processing in Motion Recognizer 302>

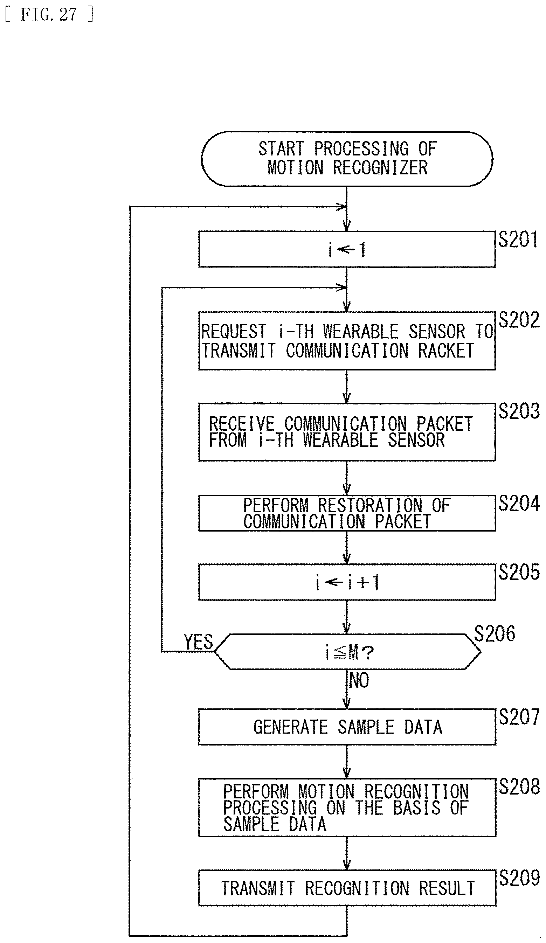

Next, with reference to a flowchart in FIG. 27, description is given of processing in the motion recognizer 302.

This processing is started, for example, when the power of the motion recognizer 302 is turned on, and is finished when the power is turned off.

In step S201, the communication control section 361 sets the value of a variable i to one.

In step S202, the motion recognizer 302 requests an i-th wearable sensor (wearable sensor 301-i) to transmit a communication packet. Specifically, the communication control section 361 generates a transmission request signal for requesting the wearable sensor 301-i to transmit a communication packet, and supplies the transmission request signal to the transmission section 362. The transmission section 362 transmits the transmission request signal to the wearable sensor 301-i.

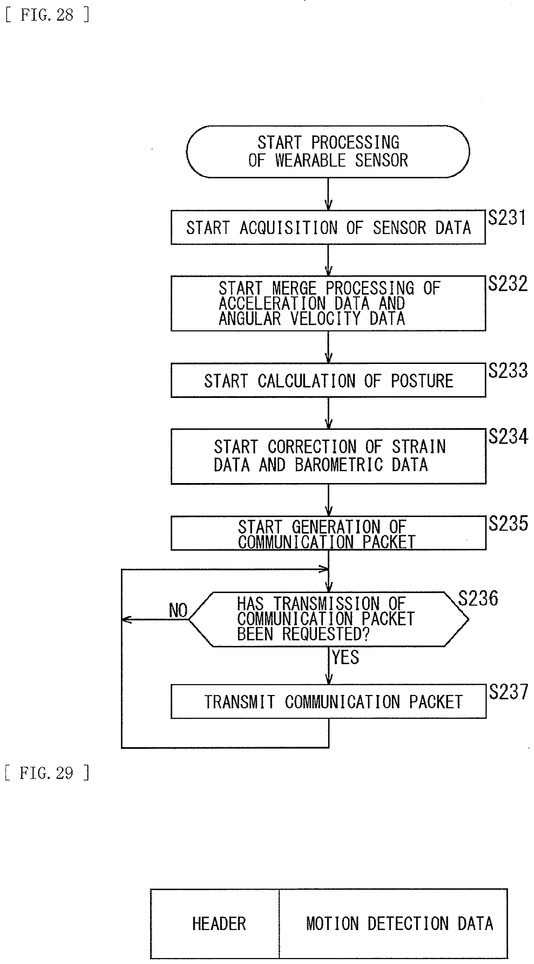

On the other hand, the wearable sensor 301-i receives the transmission request signal in step S236 in FIG. 28 described later, and transmits the communication packet in step S237.

In step S203, the reception section 161 receives the communication packet from the i-th wearable sensor (wearable sensor 301-i). The reception section 161 supplies the received communication packet to the communication control section 361. The communication control section 361 supplies the communication packet to the packet restoration section 162.

In step S204, the packet restoration section 162 restores the communication packet. For example, the packet restoration section 162 performs decoding, etc. of the motion detection data included in the acquired a communication packet, and then supplies the motion detection data to the sample data generation section 164.

In step S205, the communication control section 361 increments the value of the variable i by one.

In step S206, the communication control section 361 determines whether or not the value of the variable i is equal to or smaller than a constant M. Here, the constant M indicates the number of the wearable sensors 301, and M=6 holds true in the present example. In a case where it is determined that the value of the variable i is equal to or smaller than the constant M, i.e., in a case where there is a wearable sensor 301 that has not yet requested to transmit a communication packet, the processing returns to step S202.

Thereafter, processing in step S202 to step S206 is repeatedly executed until it is determined in step S206 that the value of the variable i exceeds the constant M. As a result, all the wearable sensors 301 are requested to transmit a communication packet in sequence, and the communication packets from the respective wearable sensors 301 are received in sequence.

It is to be noted that, for example, in parallel with the restoration of the communication packet in step S204, a subsequent wearable sensor 301 may be requested to transmit the communication packet.

On the other hand, in a case where it is determined in step S206 that the value of the variable i exceeds the constant M, the processing proceeds to step S207.

In step S207, the sample data generation section 164 generates sample data. That is, the sample data generation section 164 generates the sample data including the motion detection data from the wearable sensor 301 supplied from the packet restoration section 162. The sample data generation section 164 supplies the generated sample data to the motion recognition section 165.

Then, in steps S208 and S209, processing similar to those in steps S36 and S37 in FIG. 9 is executed. Thereafter, the processing returns to step S201, and processing from step S201 onward is executed.

### <Processing in Wearable Sensor 301>

Next, with reference to a flowchart in FIG. 28, description is given of processing executed by the wearable sensor 301 in a manner corresponding to the processing of the motion recognizer 302 in FIG. 27.

This processing is started, for example, when the power of the wearable sensor 301 is turned on, and is finished when the power is turned off.

In steps S231 to S234, processing similar to those in steps S1 to S4 in FIG. 6 is performed.

In step S235, the packet generation section 331 starts generating a communication packet. Specifically, the packet generation section 331 starts processing of reading out the latest posture data, acceleration data, angular velocity data, strain data, and barometric pressure data from the buffer 143, generating a communication packet including the read data, and accumulating the communication packet in the buffer 332.

FIG. 29 illustrates an example of a format of the communication packet generated by the packet generation section 331.

This communication packet differs from the communication packet in FIG. 7 in that the time stamp is deleted. That is, the motion recognizer 302 controls a timing at which each wearable sensor 301 transmits a communication packet, thus making the time stamp unnecessary.

In step S236, the transmission control section 334 determines whether or not transmission of the communication packet has been requested. This determination processing is repeatedly executed at a predetermined timing until it is determined that the transmission of the communication packet has been requested.

Then, in a case where the transmission control section 334 receives a transmission request signal from the motion recognizer 302 via the reception section 333, the transmission control section 334 determines that the transmission of the communication packet has been requested, and the processing proceeds to step S237.

In step S237, the wearable sensor 301 transmits a communication packet. Specifically, the transmission control section 145 reads out the communication packet from the buffer 332 and supplies the communication packet to the transmission section 146. The transmission control section 145 deletes the read communication packet from the buffer 332. The transmission section 146 transmits the communication packet to the motion recognizer 302.

Thereafter, the processing returns to step S236, and processing from step S236 onward is executed.

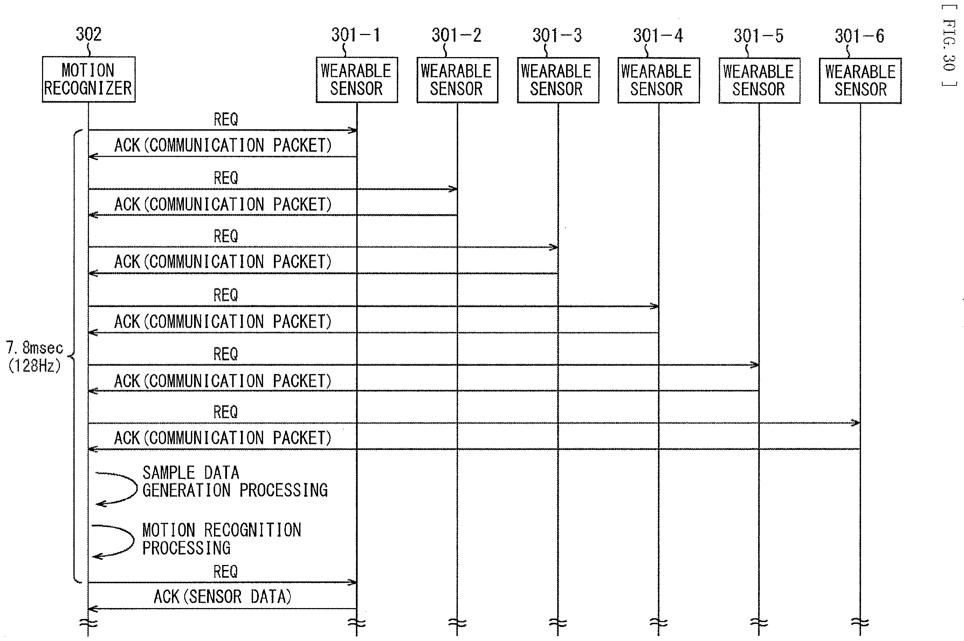

FIG. 30 is a sequence diagram illustrating communication between the motion recognizer 302 and each wearable sensor 301 in the processing of FIGs. 27 and 28.

As described above, the motion recognizer 302 transmits a transmission request signal (REQ) to each of the wearable sensors 301 in sequence, and the respective wearable sensors 301 transmit communication packets (ACK) in sequence. Accordingly, it is possible to adjust detection timings of the motion detection data of the respective wearable sensors 301 used in the motion recognition processing without using a synchronization signal, a time stamp, or the like. Accordingly, it is possible to improve the accuracy in the motion recognition processing. Further, in the motion recognizer 302, the processing of buffering and waiting the communication packet until the communication packets from the respective wearable sensors 301 are arranged becomes unnecessary.

Further, as described above with reference to FIG. 29, there is no need to add a time stamp to the communication packet, and thus a data amount of the communication packet is reduced. As a result, communication time for the communication packet is shortened, and thus the power consumption is reduced. In addition, it is possible to increase the number of retransmissions of the communication packet at the time of communication error and thus to improve the stability of communication.

After receiving the communication packet from each wearable sensor 301, the motion recognizer 302 generates a sample data, and executes the motion recognition processing. For example, in a case where the motion recognition processing is performed 128 times per second, the above processing is executed at a cycle of 7.8 milliseconds.

It is to be noted that, for example, the wearable sensor 301 may generate the communication packet and transmit the generated communication packet when receiving transmission request information from the motion recognizer 302.

### <<3. Third Embodiment>>

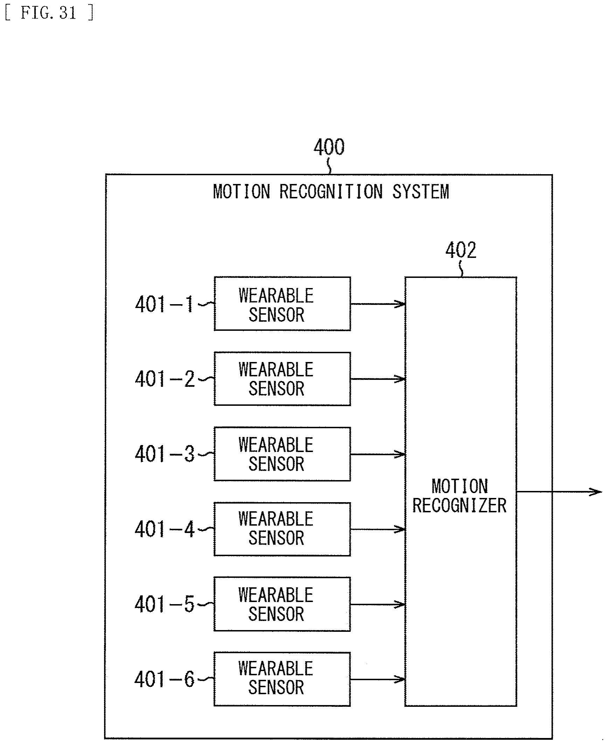

Next, with reference to FIGs. 31 to 36, description is given of a third embodiment of the present technology.

In the third embodiment, the motion recognition processing is performed using the motion detection data detected at respective detection points at the same timing as much as possible by a method different from the second embodiment.

### <Third Embodiment of Motion Recognition System>

FIG. 31 illustrates a configuration example of a motion recognition system 400 which is a third embodiment of the motion recognition system to which the present technology is applied.

The motion recognition system 400 includes a wearable sensor 401-1 to a wearable sensor 401-6, and a motion recognizer 402.

It is to be noted that, in the following, in a case where the wearable sensor 401-1 to the wearable sensor 401-6 need not be individually distinguished, they are simply referred to as a wearable sensor 401.

Similarly to the wearable sensor 101 in FIG. 1, each wearable sensor 401 is attached to a different detection point of the user, detects the movement of each detection point, and transmits a communication packet including detection results to the motion recognizer 402.

The motion recognizer 402 performs processing of the motion recognition of the user on the basis of motion detection data of each detection point included in the communication packet received from each wearable sensor 401. The motion recognizer 402 transmits the motion control data based on results of the motion recognition of the user to an apparatus in a subsequent stage.

### <Configuration Example of Wearable Sensor 401>

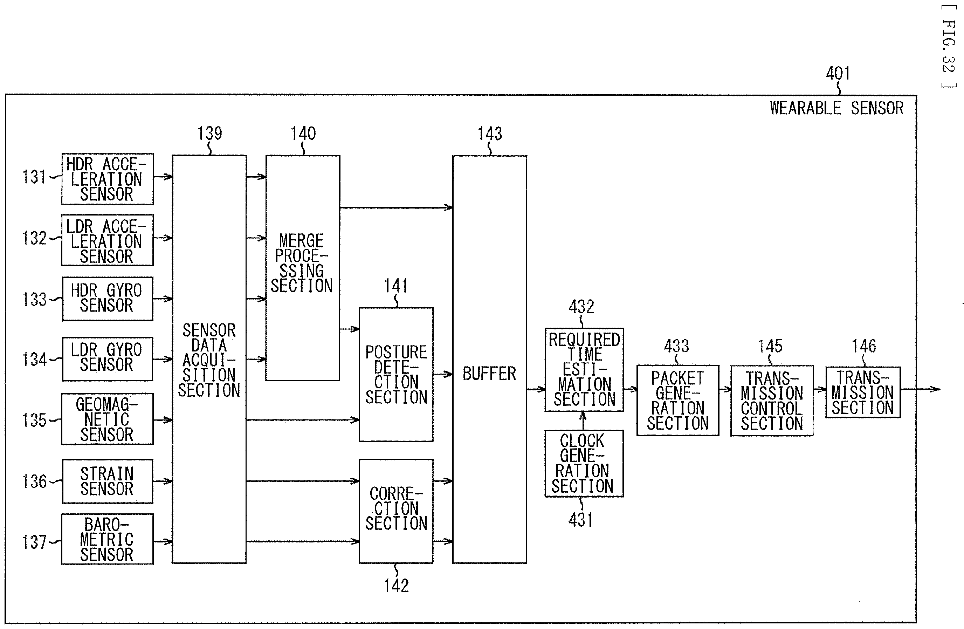

FIG. 32 illustrates a configuration example of the wearable sensor 401 in FIG. 31. In It is to be noted that, in the diagram, parts corresponding to those of the wearable sensor 101 in FIG. 3 are denoted by the same reference numerals, and descriptions thereof are omitted as appropriate.

The wearable sensor 401 differs from the wearable sensor 101 in that a packet generation section 433 is provided instead of the packet generation section 144, and a clock generation section 431 and a required time estimation section 432 are added.

The clock generation section 431 generates a clock signal of a predetermined frequency, and supplies the clock signal to each section of the wearable sensor 401 such as the required time estimation section 432. It is to be noted that, the clock generation section 431 is provided in the wearable sensor 101 in FIG. 3 and the wearable sensor 301 in FIG. 25, but illustration thereof is omitted because the illustration is not particularly necessary for description of the processing.

The required time estimation section 432 reads out, from the buffer 143, posture data, acceleration data, angular velocity data, strain data, and barometric pressure data to be transmitted to the motion recognizer 402, and supplies the pieces of data to the packet generation section 433. In addition, the required time estimation section 432 estimates time required for transmission (hereinafter, referred to as transmission required time) of the data on the basis of the clock signal, and supplies information indicating estimation results to the packet generation section 433.

Packet generation section 433 generates a communication packet for transmission of data supplied from the required time estimation section 432, and supplies the communication packet to the transmission control section 145.

### <Configuration Example of Motion Recognizer 402>

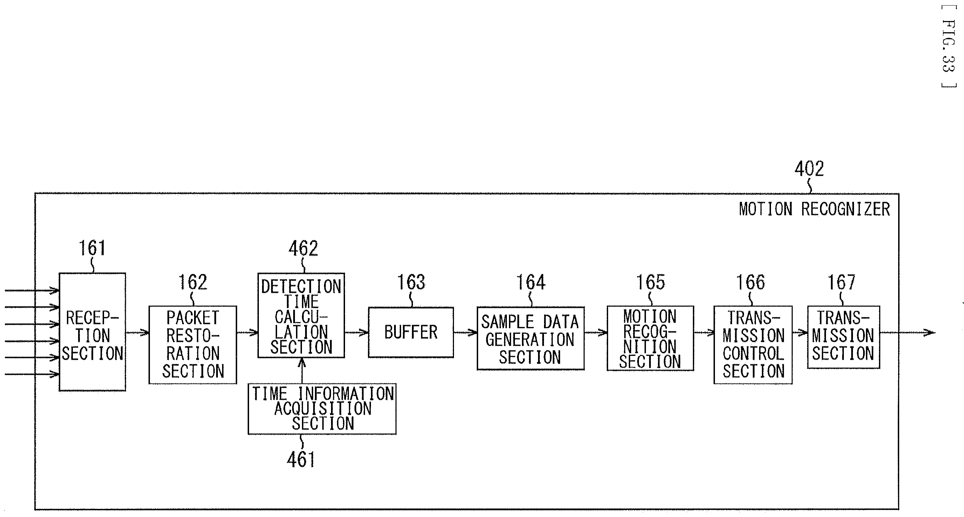

FIG. 33 illustrates a configuration example of the motion recognizer 402 in FIG. 31. It is to be noted that, in the diagram, parts corresponding to those of the motion recognizer 102 in FIG. 5 are denoted by the same reference numerals, and descriptions thereof are omitted as appropriate.

The motion recognizer 402 differs from the motion recognizer 102 in that a time information acquisition section 461 and a detection time calculation section 462 are added.

The time information acquisition section 461 is configured by, for example, a clock, etc., and acquires time information indicating the current time and supplies the acquired time information to the detection time calculation section 462.

The detection time calculation section 462 acquires, from the packet restoration section 162, the motion detection data and the transmission required time included in the communication packet received from each wearable sensor 401. Then, the detection time calculation section 462 calculates time at which the movement of a detection point corresponding to the motion detection data is detected (hereinafter, referred to as motion detection time) on the basis of the current time and the transmission required time. The detection time calculation section 462 associates the motion detection data with the motion detection time, and supplies the associated data to the buffer 163.

### <Processing in Wearable Sensor 401>

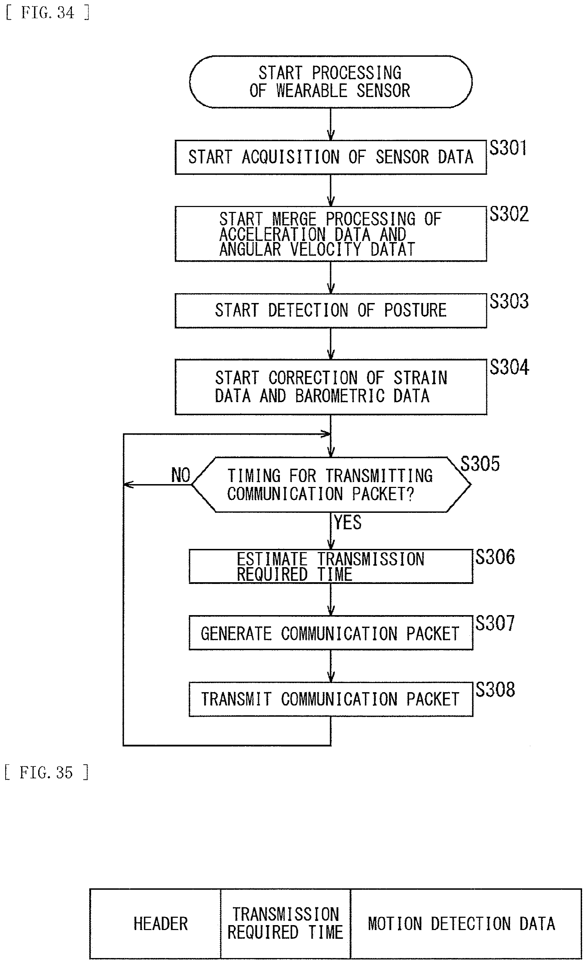

Next, with reference to the flowchart in FIG. 34, description is given of processing in the wearable sensor 401. This processing is started, for example, when the power of the wearable sensor 401 is turned on, and is finished when the power is turned off.

In steps S301 to S304, processing similar to those in steps S1 to S4 in FIG. 6 is performed.

In step S305, the required time estimation section 432 determines whether or not it is a timing to transmit a communication packet. This determination processing is repeatedly executed until it is determined to be a timing to transmit the communication packet; in a case where it is determined to be a timing to transmit the communication packet, the processing proceeds to step S306.

In step S306, the required time estimation section 432 estimates the transmission required time. Specifically, the required time estimation section 432 reads out the latest posture data, acceleration data, angular velocity data, strain data, and barometric pressure data from the buffer 143 as data to be transmitted to the motion recognizer 402. Then, the required time estimation section 432 estimates the transmission required time required for transmission of the motion detection data including such data.

The transmission required time is, for example, required time from the time when the movement of the detection point is detected to the time when the communication packet including the motion detection data corresponding to the detected movement arrives at the motion recognizer 402.

The time at which the movement of the detection point is detected (motion detection time) is, for example, the time at which detection of the movement of the detection point is started. More specifically, for example, the motion detection time is acquisition time of sensor data first acquired by the sensor data acquisition section 139, among sensor data to be used for the motion detection data. For example, as the motion detection data, sensor data of each of HDR acceleration data, LDR acceleration data, HDR angular velocity data, LDR angular velocity data, geomagnetic data, strain data, and barometric pressure data are used. Then, the earliest time among the acquisition time of each sensor data used for the motion detection data to be transmitted to the motion recognizer 402 is set as the motion detection time.

For example, the required time estimation section 432 estimates time required from the time when the movement of the detection point is detected to the time when the communication packet including the motion detection data corresponding to the detected movement is generated (hereinafter, referred to as packet generation period). The packet generation period is, for example, a time period between the time at which the detection of the movement of the detection point is started (motion detection time) and the time at which the generation of the communication packet including the motion detection data corresponding to the detected motion is completed (hereinafter, referred to as packet generation time).

For example, the required time estimation section 432 calculates the packet generation period on the basis of the size, etc. of each sensor data using a predetermined calculation formula. The calculation formula for the packet generation period is prepared in advance on the basis of, for example, a timing at which the sensor data acquisition section 139 acquires the sensor data, a processing content from the acquisition of the sensor data to the generation of the communication packet, processing capability of the wearable sensor 401, and the like.

In addition, for example, the required time estimation section 432 estimates time required from the generation of the communication packet to the arrival of the communication packet at the motion recognizer 402 (hereinafter, referred to as packet transmission time). The packet transmission time is, for example, time between the packet generation time and the time at which the communication packet arrives at the motion recognizer 402 (hereinafter, referred to as packet arrival time).

For example, the required time estimation section 432 calculates the packet transmission time on the basis of communication packet size, communication status between the wearable sensor 401 and the motion recognizer 402, and the like, using a predetermined calculation formula. The calculation formula for the packet transmission time is prepared in advance on the basis of, for example, a communication method and a communication path between the wearable sensor 401 and the motion recognizer 402, processing capability of the wearable sensor 401 and the motion recognizer 402, and the like.

It is to be noted that, for example, the packet transmission time may be calculated on the basis of an actual measurement time in a case where the communication packet is transmitted and received in advance between the wearable sensor 401 and the motion recognizer 402.

Then, the required time estimation section 432 adds the packet generation period and the packet transmission time to calculate the transmission required time.

It is to be noted that, in a case where the communication packet is retransmitted due to a communication error or the like, time required for the retransmission is also added to the transmission required time. For example, time required for detection of the communication error, waiting time until retransmission is performed, time required for retransmission processing, and the like are added to the transmission required time.

The required time estimation section 432 supplies posture data, acceleration data, angular velocity data, strain data, barometric pressure data, and estimation results of the transmission required time read out from the buffer 143 to the packet generation section 433. Further, the packet generation section 144 deletes each data transmitted by the communication packet and data older than that data from the buffer 143.

In step S307, the packet generation section 433 generates a communication packet.

FIG. 35 illustrates an example of a format of the communication packet generated by the packet generation section 433. This communication packet differs from the communication packet in FIG. 7 in that transmission required time is included instead of a time stamp. That is, the transmission required time estimated by the required time estimation section 432 is included in the communication packet.

Then, the packet generation section 433 supplies the communication packet to the transmission control section 145.

In step S308, the transmission section 146 transmits the communication packet to the motion recognizer 402 under the control of the transmission control section 145.

Thereafter, the processing returns to step S305, and processing from step S305 onward is executed.

### <Processing in Motion Recognizer 402>

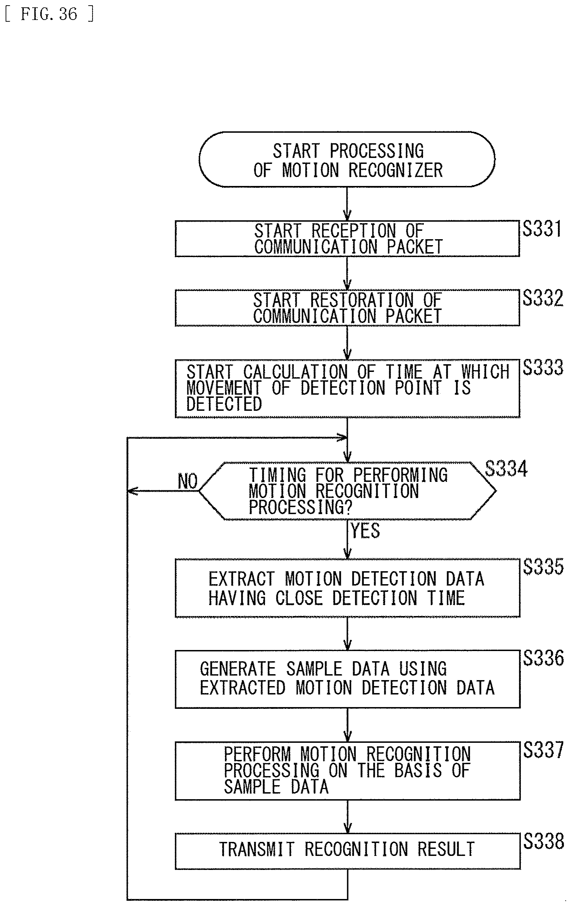

Next, with reference to a flowchart in FIG. 36, description is given of processing executed by the motion recognizer 402 in a manner corresponding to the processing of each wearable sensor 401 in FIG. 34.

This processing is started, for example, when the power of the motion recognizer 402 is turned on, and is finished when the power is turned off.

In step S331, the reception section 161 starts receiving a communication packet. That is, the reception section 161 starts processing of receiving a communication packet transmitted from each wearable sensor 401 and supplying the received communication packet to the packet restoration section 162.

In step S332, the packet restoration section 162 starts restoring the communication packet. For example, the packet restoration section 162 performs decoding, etc. of the motion detection data included in the communication packet received from each wearable sensor 101, and then starts processing of supplying the restored communication packet to the detection time calculation section 462.

In step S333, the detection time calculation section 462 starts calculating the time at which the movement of the detection point is detected. Specifically, the detection time calculation section 462 starts calculating the time (motion detection time) at which the movement of the detection point corresponding to the motion detection data included in the communication packet supplied from the packet restoration section 162 is detected. Further, the detection time calculation section 462 starts processing of associating the motion detection data with the motion detection time, and supplying the associated motion detection data to the buffer 163 for accumulation.

Here, description is given of an example of a method of calculating the motion detection time.

For example, the detection time calculation section 462 estimates the time required from the time when the communication packet is received by the reception section 161 (from the time when the communication packet arrives at the motion recognizer 402) to the time when the communication packet is restored by the packet restoration section 162 (hereinafter, referred to as packet restoration time).

For example, the detection time calculation section 462 calculates the packet restoration time on the basis of the size, etc. of the communication packet using a predetermined calculation formula. The calculation formula for the packet restoration time is prepared in advance on the basis of, for example, the content of restoration processing of the communication packet, the processing capability of the motion recognizer 402, and the like.

Then, the detection time calculation section 462 acquires time information indicating the current time from the time information acquisition section 461, and calculates the motion detection time by subtracting the transmission required time included in the communication packet and the estimated packet restoration time from the current time.

It is determined in step S334 whether or not it is a timing to perform the motion recognition processing, similarly to the processing in step S3 in FIG. 9. This determination processing is repeatedly executed at a predetermined timing; in a case where it is determined to be a timing to perform the motion recognition processing, the processing proceeds to step S335.

In step S335, the sample data generation section 164 extracts motion detection data having close detection time. That is, the sample data generation section 164 uses the motion detection time instead of the time stamp to extract the motion detection data by a method similar to the processing of step S34 in FIG. 9. As a result, pieces of motion detection data having close motion detection time are extracted one by one for the respective detection points. That is, pieces of the motion detection data corresponding to the movements detected at substantially the same timing at the respective detection points are extracted.

Then, in steps S336 to S338, processing similarto those in steps S35 to S37 in FIG. 9 is executed.

Thereafter, the processing returns to step S334, and the processing from step S334 onward is executed.

As described above, it is possible, in the motion recognizer 402, to easily and accurately detect the motion detection time. Then, the motion recognition processing is performed using the motion detection data detected at the respective detection points at the same timing as much as possible, and as a result, the motion recognition accuracy is improved.

Further, it is possible to start the communication immediately without measuring the required time or synchronizing the time after each wearable sensor 401 and the motion recognizer 402 are powered on,. Further, for example, it is possible to immediately resume the communication at a timing when the communication path is restored without measuring the required time or without synchronizing the time, even when the communication path becomes unstable and the transmission of the communication packet is temporarily stopped.

### <<4. Fourth Embodiment>>

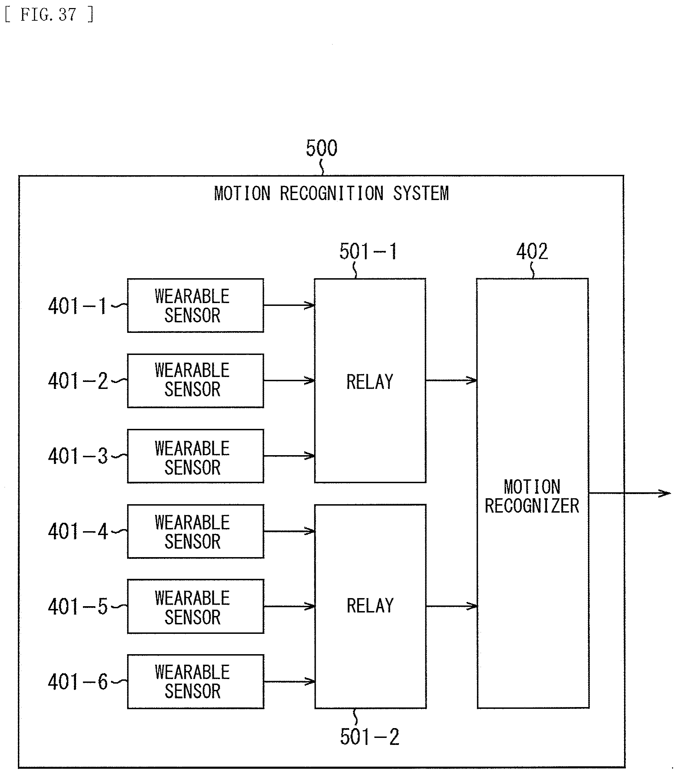

Next, with reference to FIGs. 37 to 40, description is given of a fourth embodiment of the present technology.

The fourth embodiment is an embodiment in which the wearable sensor 401 and the motion recognizer 402 communicate with each other via a relay.

### <Fourth Embodiment of Motion Recognition System>

FIG. 37 illustrates a configuration example of a motion recognition system 500 which is a fourth embodiment of the motion recognition system to which the present technology is applied.

It is to be noted that, in the diagram, parts corresponding to those of the motion recognition system 400 in FIG. 31 are denoted by the same reference numerals, and descriptions thereof are omitted as appropriate.

The motion recognition system 500 differs from the motion recognition system 400 in that a relay 501-1 and a relay 501-2 are added.

The relay 501-1 relays a communication packet between the wearable sensor 401-1 to the wearable sensor 401-3 and the motion recognizer 402. That is, the relay 501-1 receives a communication packet from the wearable sensor 401-1 to the wearable sensor 401-3, and transfers the communication packet to the motion recognizer 402.

The relay 501-2 relays a communication packet between the wearable sensor 401-4 to the wearable sensor 401-6 and the motion recognizer 402. That is, the relay 501-2 receives a communication packet from the wearable sensor 401-4 to the wearable sensor 401-6, and transfers the communication packet to the motion recognizer 402.

It is to be noted that, in the following, in a case where the relay 501-1 and the relay 501-2 need not be individually distinguished, they are simply referred to as a relay 501.

### <Configuration Example of Relay 501>

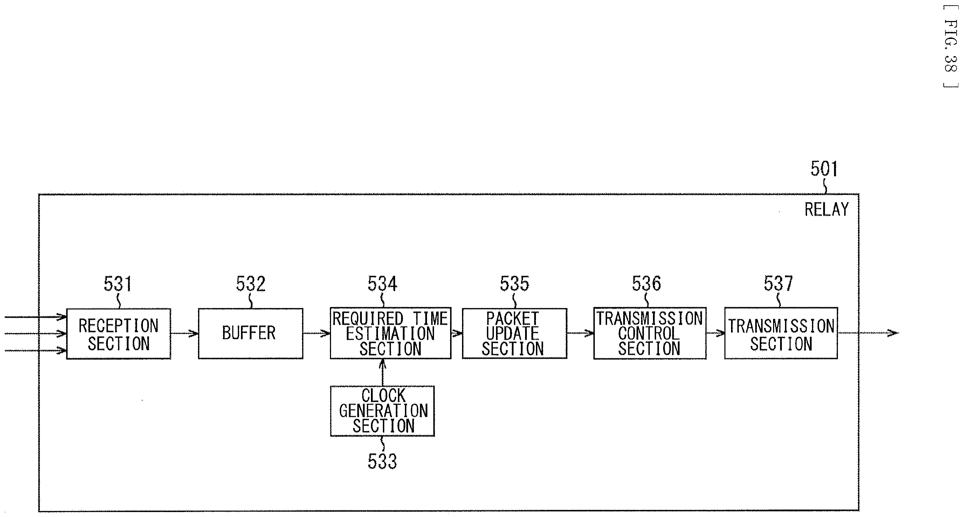

FIG. 38 illustrates a configuration example of the relay 501.

The relay 501 includes a reception section 531, a buffer 532, a clock generation section 533, a required time estimation section 534, a packet update section 535, a transmission control section 536, and a transmission section 537.

The reception section 531 receives a communication packet from each wearable sensor 401 by wireless communication of a system corresponding to the transmission section 146 of each wearable sensor 401, and supplies the communication packet to the buffer 532.

The buffer 532 temporarily accumulates communication packets.

The clock generation section 533 generates a clock signal of a predetermined frequency, and supplies the clock signal to each section of the relay 501 such as the required time estimation section 534.

The required time estimation section 534 reads out the communication packet to be transferred to the motion recognizer 402 from the buffer 532, and supplies the communication packet to the packet update section 535. In addition, the required time estimation section 432 estimates time required for relay of the communication packet (hereinafter, referred to as relay required time) on the basis of the clock signal, and supplies information indicating results of the estimation to the packet update section 535.

The packet update section 535 updates the communication packet by adding, to the communication packet, the relay required time estimated by the required time estimation section 534. The packet update section 535 supplies the updated communication packet to the transmission control section 536.

The transmission control section 536 controls transmission of the communication packet by the transmission section 537.

The transmission section 537 transmits the communication packet to the motion recognizer 402 by wireless communication of a system corresponding to the reception section 161 of the motion recognizer 402.

### <Processing in Relay 501>

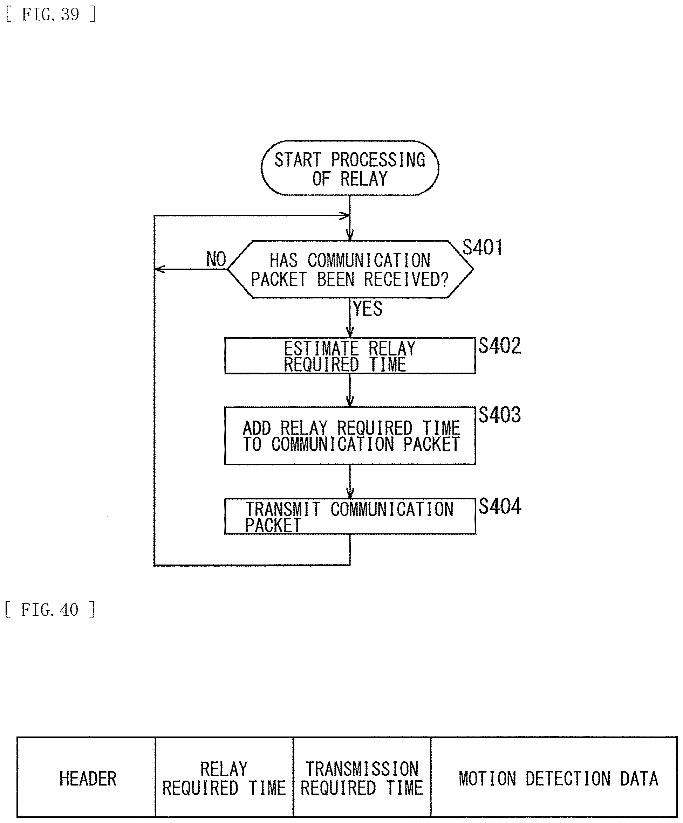

Next, with reference to a flowchart in FIG. 39, description is given of processing in the relay 501.

This processing is started, for example, when the power of the relay 501 is turned on, and is finished when the power is turned off.

It is determined in step S401 whether or not the reception section 531 has received a communication packet. This determination processing is repeatedly executed until it is determined that the communication packet has been received. Then, in a case where it is determined that the reception section 531 has received the communication packet transmitted from the wearable sensor 401, the processing proceeds to step S402. The reception section 531 accumulates received communication packets in the buffer 532.

In step S402, the required time estimation section 534 estimates the relay required time. Specifically, the required time estimation section 534 reads out the communication packet from the buffer 532. Then, the required time estimation section 534 estimates the relay required time required for relay of the communication packet.

The relay required time is, for example, required time from the time when the reception section 531 receives the communication packet (from the time when the communication packet arrives at the relay 501) to the time when the communication packet arrives at the motion recognizer 402.

For example, the required time estimation section 534 calculates the relay required time on the basis of size of the communication packet, communication status between the relay 501 and the motion recognizer 402, and the like, using a predetermined calculation formula. The calculation formula for the relay required time is prepared in advance on the basis of, for example, a communication method and a communication path between the relay 501 and the motion recognizer 402, and the processing capability of the relay 501 and the motion recognizer 402, and the like.

It is to be noted that, for example, the relay required time may be calculated in advance on the basis of the measured time in a case where the communication packet is transmitted and received between the relay 501 and the motion recognizer 402.

Further, for example, in a case where the communication packet is retransmitted due to a communication error or the like in the relay 501, the time required for the retransmission is added to the relay required time. Further, in a case where the communication packet is temporarily stored in the relay 501, the time for the storage is added to the relay required time.

The required time estimation section 534 supplies the communication packet read out from the buffer 532 and estimation results of the relay required time to the packet update section 535. Further, the required time estimation section 534 deletes the read communication packet from the buffer 532.

In step S403, the packet update section 535 adds the relay required time to the communication packet.

FIG. 40 illustrates an example of a format of the communication packet after the relay required time is added.

The communication packet in FIG. 40 differs from the communication packet in FIG. 35 in that the relay required time is added between the header and the transmission required time.

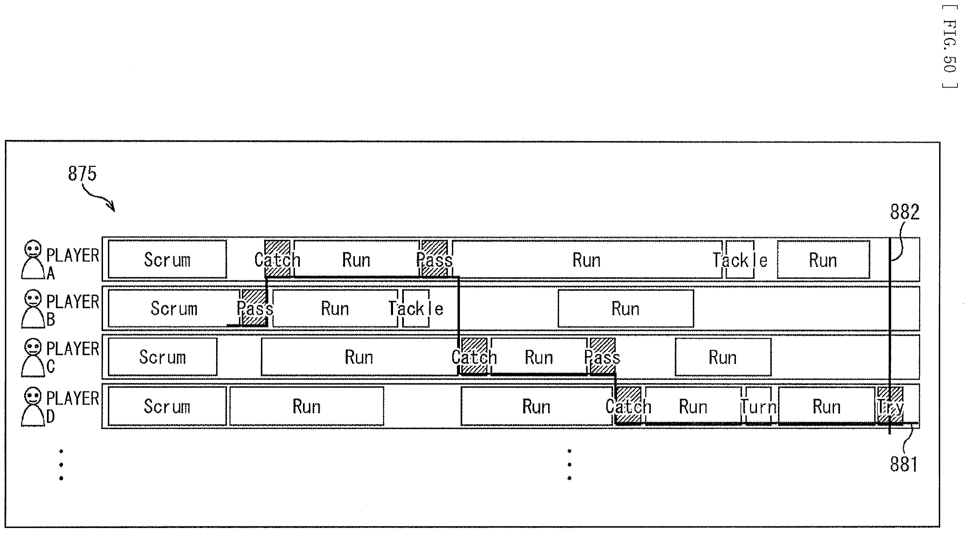

It is to be noted that, as the transmission required time of the communication packet in FIG. 50, transmission required time between the wearable sensor 401 and the relay 501 is set, instead of transmission required time between the wearable sensor 401 and the motion recognizer 402.

Then, information including the transmission required time and the relay required time constitutes the total required time information. That is, time obtained by adding the transmission required time and the relay required time constitutes the total required time from the time when the movement of detection point is detected in the wearable sensor 401 to the time when the communication packet including the motion detection data corresponding to the detected movement arrives at the motion recognizer 402.

Then, the packet update section 535 supplies the communication packet updated by the addition of the relay required time, to the transmission control section 536.

In step S404, the transmission section 537 transmits the communication packet to the motion recognizer 402 under the control of the transmission control section 536.

Thereafter, the processing returns to step S401, and processing from step S401 onward is executed.

It is to be noted that, in the above-described step S333 in FIG. 36, the detection time calculation section 462 of the motion recognizer 402 calculates the motion detection time by subtracting, from the current time, the relay required time included in the communication packet in addition to the transmission required time and the packet restoration time.

In this manner, even when the relay 501 is provided between the wearable sensor 401 and the motion recognizer 402, it is possible to detect the motion detection time easily and accurately in the motion recognizer 402.

For example, when the user wearing the wearable sensor 401 freely moves in space, a case is assumed where the communication path (relay 501) between the wearable sensor 401 and the motion recognizer 402 is dynamically altered to change communication speed and communication status. Examples of the case include a case of selecting a communication path having the best communication status, avoiding a path in which a communication error has occurred, or selecting the relay 501 closest to the wearable sensor 401.

Meanwhile, using the technique of the fourth embodiment makes it possible to start communication immediately without measuring the required time or synchronizing the time, among the wearable sensor 401, the relay 501, and the motion recognizer 402 on the communication path, even in a case where it is difficult to know a communication path to be used in advance, such as a case where the communication path is altered or a case where a plurality of communication paths are present in a meshed form. In addition, it is possible, in the motion recognizer 402 to easily and accurately detect the motion detection time of each wearable sensor 401.

Then, the motion recognition processing is performed using the motion detection data detected at the respective detection points at the same timing as much as possible, and as a result the motion recognition accuracy is improved.

It is to be noted that two or more relays 501 may be provided between the wearable sensor 401 and the motion recognizer 402. In this case, for example, the relay required time is added to the communication packet in each relay 501.

### <<5. Fifth Embodiment>

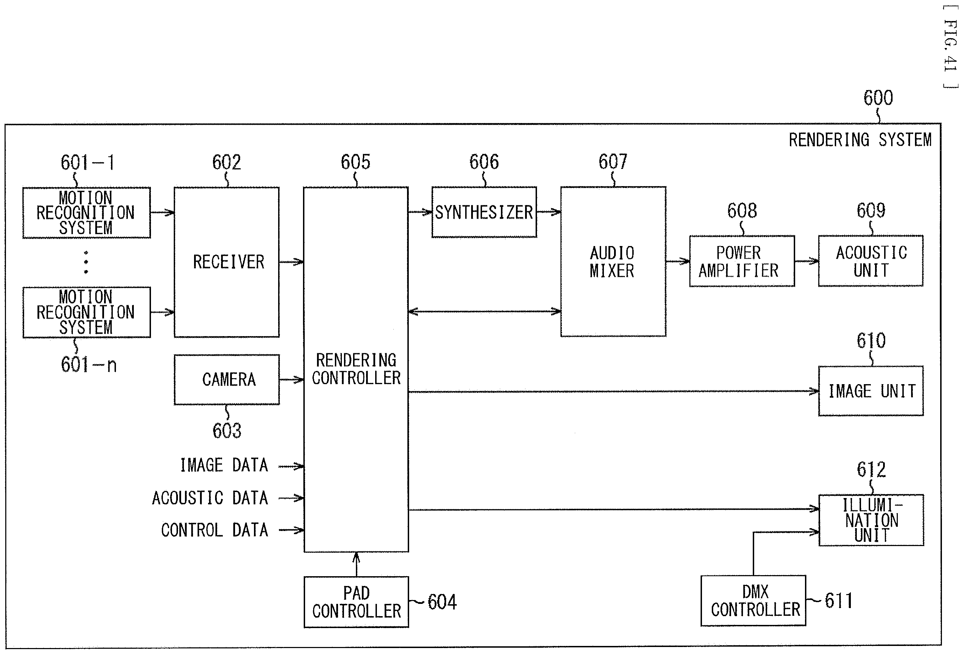

Next, with reference to FIGs. 41 to 45, description is given of a fifth embodiment of the present technology.

The fifth embodiment exemplifies a case where the above-described motion recognition system is used for rendering of various performances such as concert, dance, and theater.

### <Configuration Example of Rendering System 600>

FIG. 41 illustrates a configuration example of a rendering system 600 to which the present technology is applied.

The rendering system 600 is a system that conducts performance rendering by controlling images, sounds, illumination, and the like in accordance with the movement of each performer.

The rendering system 600 includes a motion recognition system 601-1 to a motion recognition system 601-n, a receiver 602, a camera 603, a pad controller 604, a rendering controller 605, a synthesizer 606, an audio mixer 607, a power amplifier 608, an acoustic apparatus 609, an image apparatus 610, a DMX controller 611, and an illumination apparatus 612.

It is to be noted that, in the following, in a case where the motion recognition system 601-1 to the motion recognition system 601-n need not be individually distinguished, they are referred to as a motion recognition system 601.

The motion recognition system 601 is configured by, for example, any one of the motion recognition system 100, the motion recognition system 300, the motion recognition system 400, and the motion recognition system 500 described above.

Further, the motion recognition system 601 is provided as many as the number of performers to be subjected to motion recognition, and performs motion recognition processing of each performer individually. Each motion recognition system 601 transmits, to the receiver 602, motion control data based on results of the motion recognition of each performer.

It is to be noted that, for example, in a case where there is only one performer, only one motion recognition system 601 may be used.

The receiver 602 receives the motion control data from each motion recognition system 601, and supplies the motion control data to the rendering controller 605.

It is to be noted that, for example, any system of wireless communication or wired communication is adopted for the communication between each motion recognition system 601 and the receiver 602. In addition, for example, any system of wireless communication or wired communication is adopted for the communication between the receiver 602 and the rendering controller 605.

For example, the camera 603 films each performer, stages, and the like, and supplies image data obtained as a result of the filming to the rendering controller 605.

The pad controller 604 is, for example, an input apparatus used for input of musical performance data of a keyboard, a percussion instrument, etc., and supplies the input musical performance data to the rendering controller 605. The musical performance data outputted from the pad controller 604 is, for example, data in accordance with the standard of MIDI (Musical Instrument Digital Interface).

The rendering controller 605 is configured by, for example, a personal computer, a tablet, a smartphone, and the like. It is to be noted that, in the following, description is given by exemplifying a case where the rendering controller 605 is configured by the information processor 220 illustrated in FIG. 14.

In addition to the above-described data, for example, image data, acoustic data, and control data, etc. indicating effect patterns, etc. of images, sounds, or illumination, which are used for performance rendering, are inputted to the rendering controller 605.

For example, the rendering controller 605 executes various types of processing related to the performance rendering by executing software for VJ (Video Jockey). For example, the rendering controller 605 performs image remixing, image and audio effect processing, audio analysis, BPM (Beats Per Minute) detection, and the like. In addition, for example, the rendering controller 605 performs processing and output, etc. of MIDI data, OSC (Open Sound Control) data, and DMX (Digital Multiplexing) data.

Then, the rendering controller 605 supplies sound control data for generation of sounds for rendering to the synthesizer 606, and supplies acoustic data for outputting of the sounds for rendering to the audio mixer 607. In addition, the rendering controller 605 supplies image data for outputting of images for rendering to the image apparatus 610. Further, the rendering controller 605 supplies the DMX data for controlling of the illumination for rendering to the illumination apparatus 612.

The synthesizer 606 generates various sounds for rendering on the basis of acoustic control data from the rendering controller 605, and supplies acoustic data for outputting of the generated sounds to the audio mixer 607.

The audio mixer 607 mixes the acoustic data from the rendering controller 605 and the acoustic data from synthesizer 606, and supplies the mixed acoustic data to the power amplifier 608. The audio mixer 607 supplies audio data inputted via an unillustrated microphone to the rendering controller 605.

The power amplifier 608 amplifies the acoustic data, and supplies the amplified acoustic data to the acoustic apparatus 609.

The acoustic apparatus 609 is configured by, for example, a speaker or the like, and outputs sound based on the acoustic data.

The image apparatus 610 is configured by, for example, a projector, a monitor, or the like, and projects or displays an image based on the image data, onto a stage or the like.

The DMX controller 611 is an apparatus for controlling of the illumination apparatus 612. The DMX controller 611 generates DMX data for controlling of the illumination apparatus 612 in accordance with user input or the like, and supplies the DMX data to the illumination apparatus 612.

The illumination apparatus 612 is configured by, for example, various lights, light sources, or the like. The illumination apparatus 612 performs various types of illumination rendering on the basis of the DMX data from the rendering controller 605 and the DMX controller 611.

### <Configuration Example of Rendering Control Unit 631>

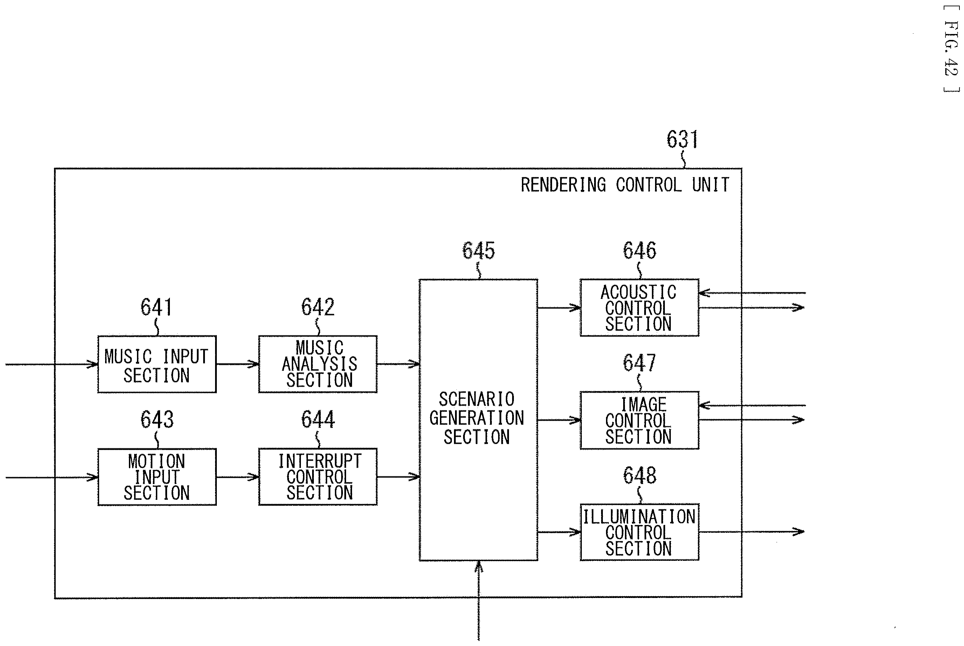

FIG. 42 illustrates a configuration example of a rendering control unit 631 achieved by the CPU 221 of the information processor 220 executing a predetermined control program in a case where the rendering controller 605 is configured by the information processor 220 in FIG. 14.

The rendering control unit 631 controls the performance rendering on the basis of music and movements of each performer. For example, in a case where a plurality of performers dance to music, the rendering control unit 631 controls the performance rendering on the basis of the music and the dance of each performer.

The rendering control unit 631 includes a music input section 641, a music analysis section 642, a motion input section 643, an interrupt control section 644, a scenario generation section 645, an acoustic control section 646, an image control section 647, and an illumination control section 648.

The music input section 641 acquires music data inputted from the outside, and supplies the music data to the music analysis section 642.

The music analysis section 642 performs analysis processing of the music data. The music analysis section 642 supplies the music data and analysis results to the scenario generation section 645.

The motion input section 643 acquires, from the receiver 602, the motion control data transmitted from each motion recognition system 601, and supplies the motion control data to the interrupt control section 644.

The interrupt control section 644 performs interrupt control on the basis of the motion control data from each motion recognition system 601. For example, in a case where it is detected that each performer has performed a predetermined motion, the interrupt control section 644 notifies the scenario generation section 645 that the motion has been detected, and adds rendering corresponding to the motion of each performer to a rendering scenario based on the analysis results of the music data.

The scenario generation section 645 generates a rendering scenario for the performance rendering on the basis of the control data supplied from the outside, the analysis results of the music data, and results of motion recognition of each performer. The scenario generation section 645 supplies the generated rendering scenarios to the acoustic control section 646, the image control section 647, and the illumination control section 648. In addition, the scenario generation section 645 supplies the music data to the acoustic control section 646.

The acoustic control section 646 process the music data supplied from the scenario generation section 645 and the acoustic data supplied from the outside on the basis of the rendering scenario, generates acoustic data for rendering, and supplies the acoustic data to the audio mixer 607. Further, the acoustic control section 646 generates the acoustic control data based on the rendering scenario, and supplies the acoustic control data to synthesizer 606.

The image control section 647 processes image data supplied from the camera 603 and image data supplied from the outside on the basis of the rendering scenario, and generates rendering image data. The image control section 647 supplies the image data to the image apparatus 610.

The illumination control section 648 generates DMX data for rendering on the basis of the rendering scenario, and supplies the DMX data for rendering to the illumination apparatus 612.

It is to be noted that, for example, achieving the rendering control unit 631 in FIG. 42 by plug-in software makes it possible to construct a rendering system with the motion recognition system 601 by using an existing rendering apparatus installed in a stage such as a hall or a live house. In addition, it is possible to simulate an actual stage environment using, for example, a personal computer, a tablet, or the like.

### <Rendering Control Processing>

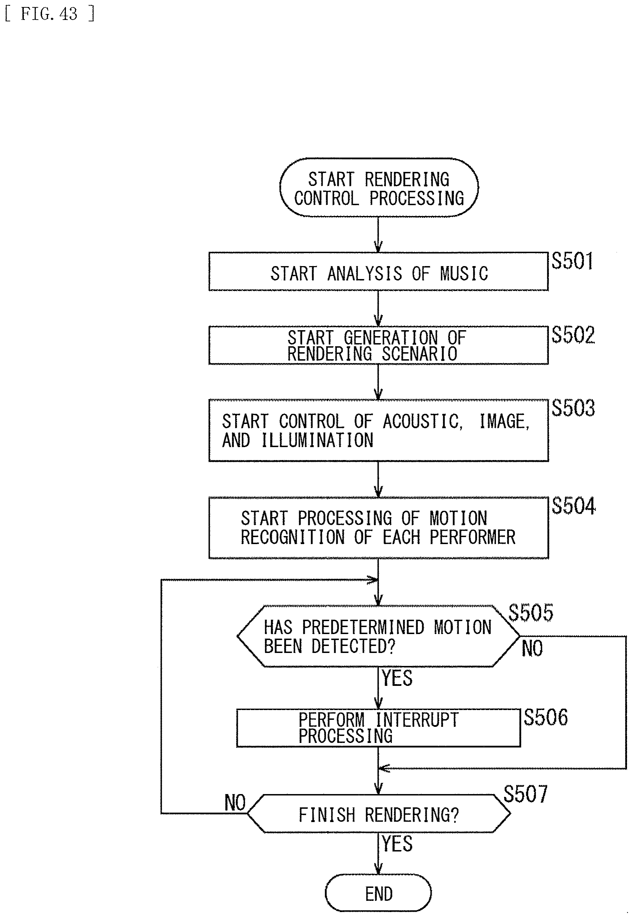

Next, with reference to a flowchart in FIG. 43, description is given of rendering control processing to be executed by the rendering system 600.

This processing is started, for example, when a command to execute performance rendering is inputted via the input unit 226 of the information processor 220 (rendering controller 605).

In step S501, the rendering control unit 631 starts analyzing music.

Specifically, the music input section 641 starts processing of acquiring music data inputted from the outside and supplying the music data to the music analysis section 642.

The music analysis section 642 starts processing of analyzing chord progression, tempo, beat, structure, instrument sounds included, and the like of music on the basis of the music data and supplying analysis results to the scenario generation section 645.

In step S502, the scenario generation section 645 starts generating a rendering scenario. Specifically, the scenario generation section 645 starts processing of controlling acoustic, image, and illumination in accordance with the music on the basis of the analysis results of the control data and the music data supplied from the outside, generating rendering scenario for conducting the performance rendering, and supplying the rendering scenario to the acoustic control section 646, the image control section 647, and the illumination control section 648. In addition, the scenario generation section 645 starts processing of supplying music data to the acoustic control section 646.

In step S503, the rendering control unit 631 starts controlling acoustic, image, and illumination.

Specifically, the acoustic control section 646 starts processing of processing the music data supplied from the scenario generation section 645 and the acoustic data supplied from the outside on the basis of the rendering scenario, generating acoustic data for rendering, and supplying the acoustic data for rendering to the audio mixer 607. Further, the acoustic control section 646 starts processing of generating acoustic control data on the basis of the rendering scenario and supplying the acoustic control data to the synthesizer 606.

The synthesizer 606 starts processing of generating various sounds for rendering on the basis of the acoustic control data and supplying acoustic data for outputting of the generated sounds to the audio mixer 607.

The audio mixer 607 starts processing of mixing the acoustic data from the rendering controller 605 and the acoustic data from the synthesizer 606, and supplying the mixed acoustic data to the power amplifier 608.

The power amplifier 608 starts processing of amplifying the acoustic data and supplying the amplified acoustic data to the acoustic apparatus 609.

The acoustic apparatus 609 starts processing of outputting sound on the basis of the acoustic data.

The image control section 647 starts processing of processing the image data supplied from the camera 603 and the image data supplied from the outside on the basis of the rendering scenario, generating rendering image data, and supplying the rendering image data to the image apparatus 610.

The image apparatus 610 starts processing of projecting or displaying an image based on the image data onto a stage or the like.

The illumination control section 648 starts processing of generating DMX data for rendering on the basis of the rendering scenario and supplying the DMX data to the illumination apparatus 612.

The DMX controller 611 starts processing of generating the DMX data in accordance with user input or the like and supplying the DMX data to the illumination apparatus 612.

The illumination apparatus 612 starts processing of performing various types of illumination rendering on the basis of the DMX data from the illumination control section 648 and the DMX controller 611.

In step S504, each motion recognition system 601 starts processing of motion recognition of each performer. Then, each motion recognition system 601 starts transmitting the motion control data based on results of the motion recognition of each performer to the receiver 602.

The receiver 602 starts processing of receiving the motion control data from each motion recognition system 601 and transmitting the motion control data to the communication unit 229 (FIG. 14) of the information processor 220 (rendering controller 605).

The communication unit 229 starts processing of supplying the motion control data of each motion recognition system 601 to the motion input section 643.

The motion input section 643 starts processing of supplying the motion control data of each motion recognition system 601 to the interrupt control section 644.

In step S505, the interrupt control section 644 determines whether or not a predetermined motion has been detected on the basis of the motion control data of each motion recognition system 601. In a case where it is determined that a predetermined motion has been detected, the processing proceeds to step S506.

In step S506, the rendering control unit 631 performs interrupt processing.

For example, the interrupt control section 644 notifies the scenario generation section 645 that a predetermined motion has been detected.

The scenario generation section 645 adds rendering corresponding to the detected motion to the rendering scenario generated on the basis of the control data supplied from the outside and analysis results of the music data.

As a result, for example, the image and the illumination are controlled in accordance with dance steps of the performer. For example, color and brightness of the illumination are controlled in accordance with rhythm of the whole body of the performer, intensity of the movement, slowness and quickness of limbs, and the like. For example, a CG character that performs the same dance technique as the performer is displayed, or illumination blinks in accordance with dance steps of the performer.

Thereafter, the processing proceeds to step S507.

On the other hand, in a case where it is determined in step S505 that a predetermined motion has not been detected, the processing of step S506 is skipped, and the processing proceeds to step S507.

In step S507, the scenario generation section 645 determines whether or not to finish the rendering. In a case where it is determined that the rendering is not finished, the processing returns to step S505.

Thereafter, the processing of steps S505 to step S507 is repeatedly executed until it is determined in step S507 that the rendering is to be finished.

On the other hand, in step S507, in a case where a command to finish the performance rendering is inputted via the input unit 226 of the information processor 220 (rendering controller 605), the scenario generation section 645 determines that the rendering is to be finished, and the rendering control processing is finished.

As described above, it is possible to perform rendering in accordance with the motion of the performer while performing the rendering based on the analysis results of the music data and the control data from the outside.

### <Specific Example of Performance Rendering>

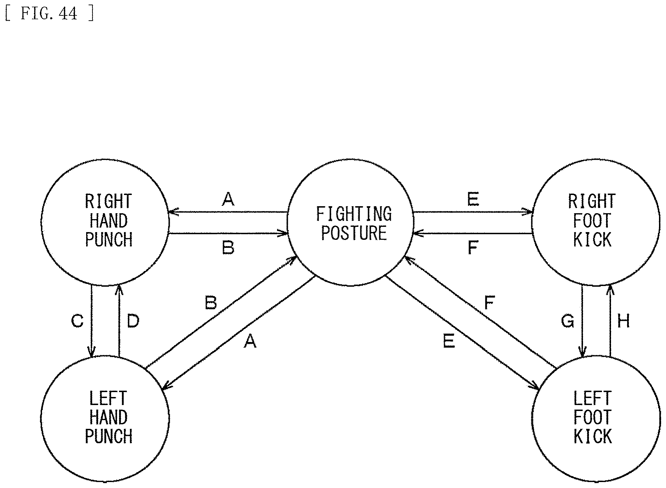

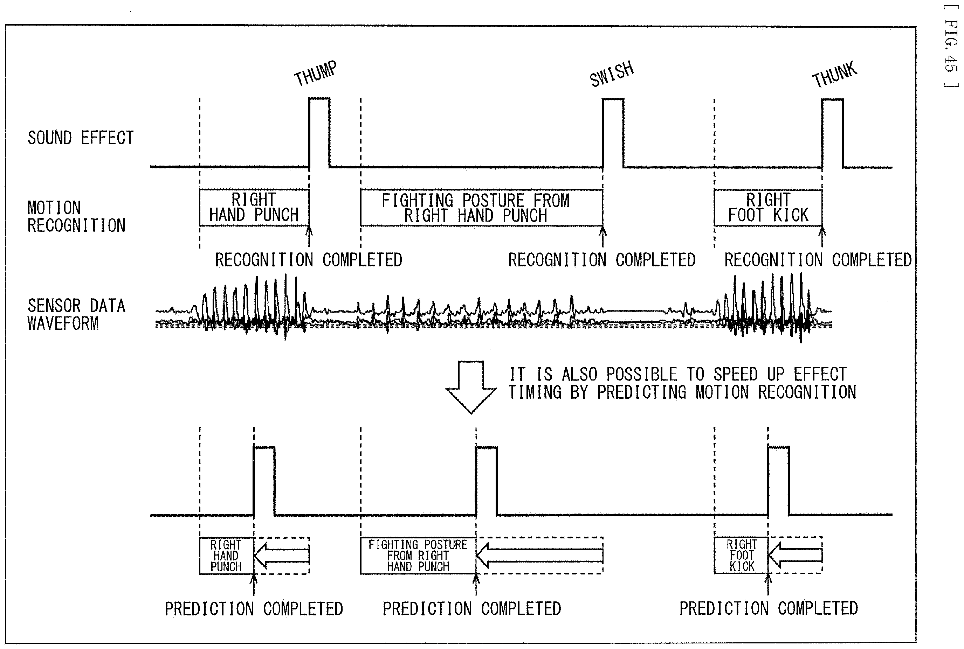

Here, with reference to FIGs. 44 and 45, description is given of a specific example of performance rendering by the rendering system 600. Specifically, description is given of an example of a case where a sound effect is outputted in accordance with styles of Karate performed by a Karate player (performer).

FIG. 44 is a transition diagram illustrating a relationship between motions (Karate techniques) to be recognized and sound effects to be outputted .

For example, a fighting posture, right hand punch, left hand punch, right foot kick, and left foot kick are assumed to be motions to be recognized.

Then, the sound effect is selected and outputted on the basis of motion transition. That is, rendering content (sound effect) is selected and outputted on the basis of combination of pre-transition motion and post-transition motion.

For example, in a case where the right hand punch or the left hand punch is performed from the state of a fighting posture the player maintains, a sound effect A is outputted; in a case where the right hand kick or the left hand kick is performed from the state of the fighting posture the player maintains, a sound effect E is outputted.

In a case where the player returns to the fighting posture after performing the right hand punch, a sound effect B is outputted; in a case where the player performs the left hand punch after performing the right hand punch, a sound effect C is outputted. In a case where the player returns to the fighting posture after performing the left hand punch, a sound effect B is outputted; and in a case where the player performs the right hand punch after performing the left hand punch, a sound effect D is outputted.

In a case where the player returns to the fighting posture after performing the right foot kick, a sound effect F is outputted; in a case where the player performs the left foot kick after performing the right foot kick, a sound effect G is outputted. In a case where the player returns to the fighting posture after performing the left foot kick, a sound effect F is outputted; and in a case where the player performs the right foot kick after performing the left foot kick, a sound effect H is outputted.

This enables the sound effect to be dynamically changed on the basis of motion transition within a series of movements of the player. As a result, it is possible to perform more effective rendering. For example, rendering with finer granularity is achieved, thus making it possible to achieve rendering that more matches the movement of the player as well as rendering with higher realistic sensation.

FIG. 45 illustrates examples of timings at which sound effect is outputted.

The upper diagram in FIG. 45 illustrates examples of time-series transitions of sound effects, motion recognition, and sensor data waveforms.

In this example, the sound effect is outputted at a time point when the player motion is completed and the motion recognition thereof is completed. For example, the player performs the right hand punch, and the right hand punch is completed; at a time point when the recognition thereof is completed, a sound effect "thump" is outputted. Next, the player returns to the fighting posture from the state where the right hand punch is performed, and the fighting posture is completed; at a time point when the recognition thereof is completed, a sound effect "swish" is outputted. Next, the player performs the right foot kick from the state of the fighting posture the player maintains, and the right foot kick is completed; at a time when the recognition thereof is completed, a sound effect "thunk" is outputted.

Meanwhile, a case is also assumed where the sound effect is desired to be outputted prior to the completion of the motion, i.e., in the middle of the motion. In contrast, for example, as illustrated in the lower diagram in FIG. 45, the motion recognition may be predicted in the motion recognition section 165 of the motion recognition system 601 to accelerate an effect timing (i.e., sound effect output timing) on the basis of results of the prediction.

For example, while the player is performing the right hand punch, the sound effect "thump" is outputted at the time point when the prediction of the recognition of the right hand punch is completed. Next, while the player is returning to the fighting posture from the state where the player has performed the right hand punch, at the time point when the prediction of the recognition of the fighting posture is completed, the sound effect "swish" is outputted. Next, while the player is performing the right foot kick from the state of the fighting posture the player maintains, at the time point when the prediction of the right foot kick is completed, the sound effect "thunk" is outputted.

In this manner, by using the prediction of the motion recognition, it is possible to perform rendering on the motion being executed at the middle stage before the motion is completed.

It is to be noted that it is also possible to control a rendering content other than the sound effect on the basis of motion transition of the user.

Further, for example, the rendering content such as a sound effect may be controlled for each type of motions instead of the motion transition. For example, the sound effect A may be outputted in a case where the player performs the right hand punch; the sound effect B may be outputted in a case where the player performs the left hand punch; the sound effect C may be outputted in a case where the player performs the right foot kick; and the sound effect D may be outputted in a case where the player performs the left foot kick.

### <<6. Sixth Embodiment>>

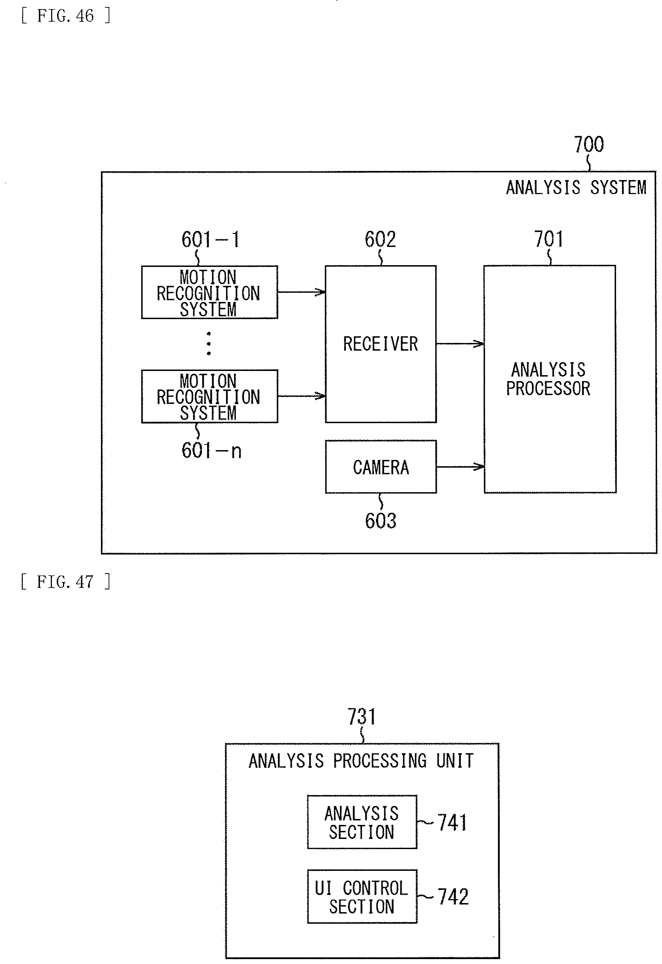

Next, with reference to FIGs. 46 to 52, description is given of a sixth embodiment of the present technology.

The sixth embodiment exemplifies a case where the above-described motion recognition system is used for analysis processing of actions of a person.

### <Configuration Example of Analysis System 700>

FIG. 46 illustrates a configuration example of an analysis system 700 to which the present technology is applied. It is to be noted that, in the diagram, parts corresponding to those of the rendering system 600 in FIG. 41 are denoted by the same reference numerals, and descriptions thereof are omitted as appropriate.

The analysis system 700 is a system that analyzes actions of one or more users.

The analysis system 700 includes the motion recognition system 601-1 to the motion recognition system 601-n, the receiver 602, the camera 603, and an analysis processor 701.

The number of motion recognition system 601 to be provided is equal to the number of users whose actions are to be analyzed. It is to be noted that, in a case where only one user is an analysis target, only one motion recognition system 601 may be used.

The analysis processor 701 is configured by, for example, a personal computer, a tablet, a smartphone, and the like. It is to be noted that, in the following, description is given by exemplifying a case where the analysis processor 701 is configured by the information processor 220 illustrated in FIG. 14.

The analysis processor 701 performs analysis processing of actions of each user on the basis of the motion control data of each motion recognition system 601 supplied from the receiver 602 and the image data supplied from the camera 603.

### <Configuration Example of Analysis Processing Unit 731>

FIG. 47 illustrates a configuration example of an analysis processing unit 731 which is achieved by the CPU 221 of the information processor 220 executing a predetermined control program in a case where the analysis processor 701 is configured by the information processor 220 in FIG. 14.

The analysis processing unit 731 includes an analysis section 741 and a UI (User Interface) control section 742.

The analysis section 741 analyzes the actions of each user on the basis of the motion control data of each motion recognition system 601 supplied from the receiver 602 and the image data supplied from the camera 603.

The UI control section 742 controls the output unit 227 on the basis of user input or the like inputted via the input unit 226 of the information processor 220 to control a user interface at the time of presenting analysis results of the actions of each user.

### <Analysis Processing>

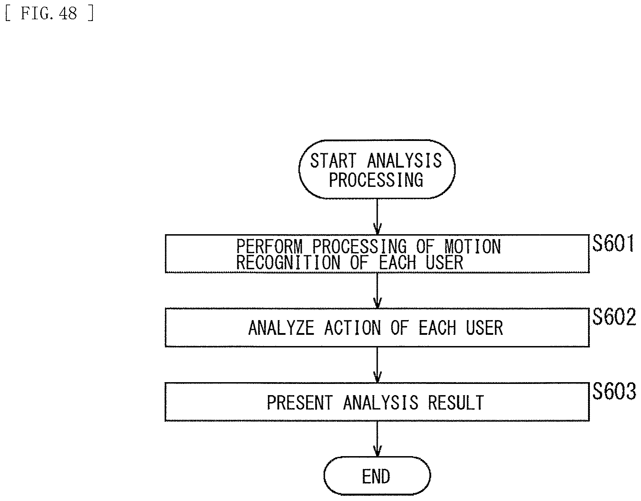

Next, with reference to a flowchart in FIG. 48, description is given of analysis processing to be executed by the analysis system 700.

This processing is started, for example, when a command to execute the analysis processing is inputted via the input unit 226 of the information processor 220 (analysis processor 701).

In step S601, each motion recognition system 601 performs processing of motion recognition of each user. Then, each motion recognition system 601 transmits motion control data based on results of the motion recognition of each user to the receiver 602.

The receiver 602 receives the motion control data from each motion recognition system 601, and transmits the motion control data to the communication unit 229 of the information processor 220 (FIG. 14).

The communication unit 229 causes the storage unit 228 to store the received motion control data of each user.

In addition, the camera 603 films behavior of each user acting, and causes the storage unit 228 to store the resulting image data.

In step S602, the analysis section 741 analyzes actions of each user. Specifically, the analysis section 741 analyzes the action of each user on the basis of the motion control data of each user stored in the storage unit 228 and the image data filmed by the camera 603. The analysis section 741 causes the storage unit 228 to store analysis data indicating analysis results.

It is to be noted that this analysis processing may be performed in parallel with the recognition processing of the motion of each user, or may be performed after the action of each user is finished and the recognition processing of the motion of each user is finished.

In step S603, the output unit 227 presents the analysis results on the basis of the analysis data stored in the storage unit 228 under the control of the UI control section 742.

Thereafter, the analysis processing is finished.

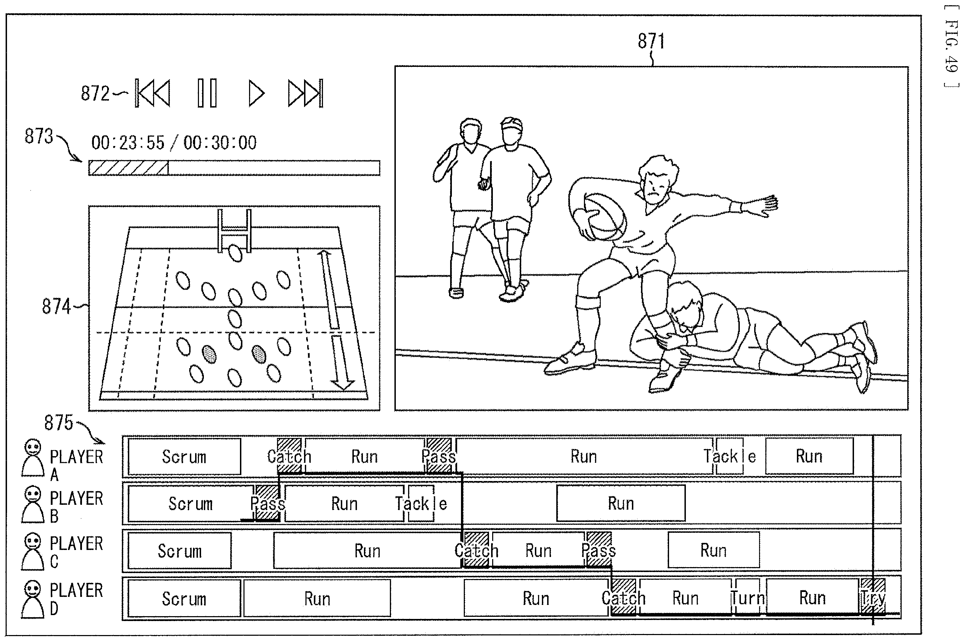

Here, with reference to FIGs. 49 to 52, description is given of an example of a method of presenting the analysis results. Specifically, description is given of an example of a case of presenting analysis results of actions of each player of a rugby match.

In this case, for example, rugby-specific movements of each player, such as stay, walk, run, pass, kick, catch, tackle, scrum, turn, and try are recognized as motions.

Then, for example, an analysis screen as illustrated in FIG. 49 is displayed in the output unit 227. The analysis screen is roughly divided into an image display part 871, an operation part 872, a time display part 873, a position analysis part 874, and an action analysis part 875.

An image of a rugby match filmed by the camera 603 is displayed in the image display part 871.

The operation part 872 displays buttons and the like for performing operations such as reproduction, stop, fast-forward, rewind, and the like, for an image displayed in the image display part 871.

The time display part 873 displays information about time of the image displayed in the image display part 871.

The position analysis part 874 displays analysis results of positions of respective players at the time of the image displayed in the image display part 871.

It is to be noted that positions (position information) of the respective players are detected using, for example, image data filmed by the camera 603, the GNSS receiver, and the like.

The action analysis part 875 displays analysis results of actions of the respective players. FIG. 50 is an enlarged view of displayed contents of the action analysis part 875.

Specifically, results of the motion recognition of a plurality of players are displayed in an aligned manner in the vertical direction in the action analysis part 875. For example, in the example in FIG. 50, results of the motion recognition of a player A to a player D are displayed in an aligned manner in the vertical direction. For example, a player to be displayed may be changed by scrolling the display in the vertical direction in the action analysis part 875.

In addition, transitions of the motions (movements) for each player are displayed in a time series in the horizontal direction. For example, it is illustrated that motions of the player A are transitioned in the order of scrum, catch, run, pass, run, tackle, and run.

In addition, among the motions of each player, motions relating to move of the ball such as pass, catch, and try, and scoring, are displayed in different colors, etc. to be distinguishable from other motions.

Further, the position of the ball is tracked on the basis of the analysis results of the actions of each player, and a result thereof is displayed by a tracking line 881.

Further, a time line 882 is displayed that corresponds to the time of the image displayed in the image display part 871. By sliding the time line 882 left and right, it is possible to adjust the time of the image displayed in the image display part 871. This makes it possible to easily search the image at the time when each player performs the motions.

As described above, the analysis screens of FIGs. 49 and 50 make it possible to grasp and analyze, in detail, the motions of each player, combination between players, and the like, which are unknown only by the image.

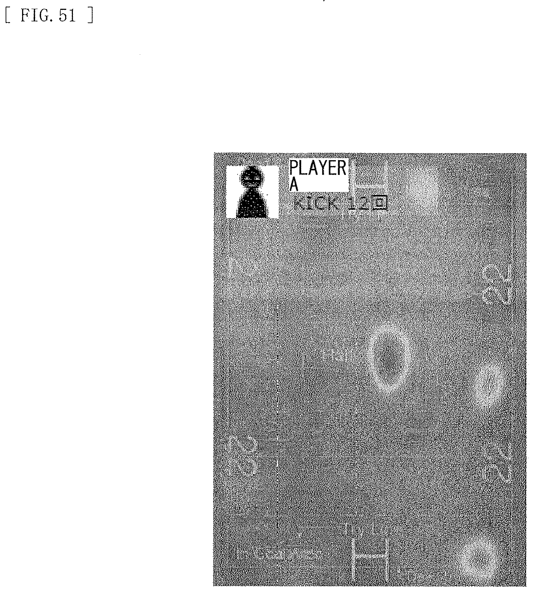

FIG. 51 illustrates an example in which analysis results of executing positions of the motions of each player are presented on the basis of results of the motion recognition and the position information of each player. Specifically, the number of times of kicking by the player A is displayed on the analysis screen in FIG. 51, and the position at which the kick has been performed is displayed in the form of a heat map. For example, a region in which the number of times of kicking by the player A is large is highlighted and displayed.

On the analysis screen in FIG. 51, for example, players to be analyzed may be switched, or motions to be analyzed may be switched. Further, for example, a plurality of players may be set to be analyzed all at once, or a plurality of motions may be set to be analyzed all at once.

This makes it possible to grasp the relationship between the motions of each player and the executing positions of the motions in detail.

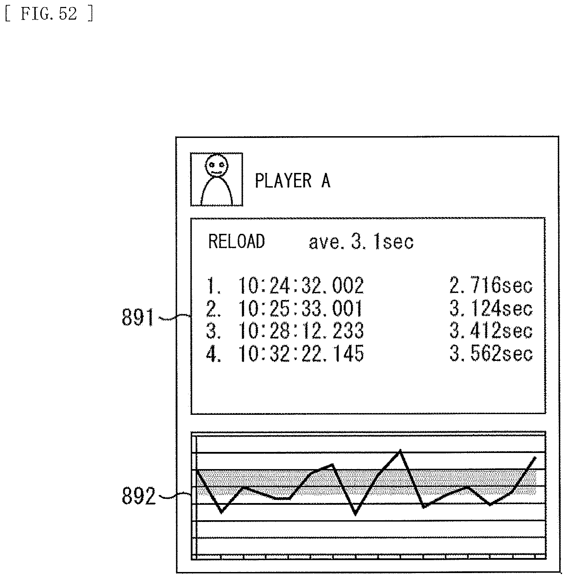

FIG. 52 illustrates an example in which results are presented in which time required for reloading of each player (e.g., time from falling to returning to the play of a player) is analyzed on the basis of the results of the motion recognition of each player.

Specifically, a window 891 in FIG. 52 displays a list of time at which the player A has performed reloading and the time required for the reloading.

In addition, a window 892 illustrates a line graph indicating a change in time required for the reloading. In the graph, the horizontal axis indicates the number of reloading, i.e., how many times the reloading has been performed, and the vertical axis indicates the time required for each reloading.

It is to be noted that, in the example described above, the description has been given of the instance in which the analysis results of the rugby match are presented; however, it is needless to say that the present technology is applicable to other sports. For example, the heat map in FIG. 51 is applicable to a position at which a soccer ball is kicked, a position at which a basket ball is shot, a position at which a hand ball is passed, and the like.

### <<7. Application Example>>

Next, description is given of an application example of the present technology other than the above-described examples.

### <Application Example of Motion Recognition Processing>

For example, it is possible to perform operation of various apparatuses, input of commands, and the like by a motion recognized by the motion recognition processing of the present technology.

Specifically, it is possible to operate, for example, various apparatuses as well as a system using VR (Virtual Reality) or AR (Augmented Reality).

For example, in a virtual fighting game, it is possible to use the motion recognition processing of the present technology for recognition of a technique of the player. Then, for example, in a case where the recognized technique hits an opponent player, a vibration may be fed back to a system of the attacking player.

For example, in a game using AR, in a case where a motion of a user throwing a ball is recognized, a point at which the ball hits may be calculated in accordance with the speed or direction, and an item at the point may be acquired.

For example, in a system using AR, in a case where a motion of opening a door is recognized, a displayed door may be opened, or in a case where a motion of opening a box is recognized, a displayed box may be opened.

For example, a drone or the like may be operated by a motion of a recognized user.

Further, for example, it is possible to apply the motion recognition processing of the present technology to evaluation of movements of sports or the like.

Specifically, for example, in a dance school or a yoga school, it is possible to compare results of motion recognition of a teacher and a student to feed back the comparison results to the student in real time by sound, vibration, or the like. This enables, for example, the student to immediately recognize whether or not his or her movement or pose is correct even when the student is in a position where the teacher is not visible. It is to be noted that, in this case, the motion recognition accuracy may be changed depending on levels of the class. For example, the recognition accuracy may be tightened to allow each motion to be accurately recognized in an advanced class, while the recognition accuracy may be relaxed in a beginner's class to allow a plurality of motion candidates to be recognized for one movement of the user.

Further, for example, on the basis of results of motion recognition of the performer, it is possible to automatically generate a promotional video by clipping and connecting parts where specific techniques are performed in the image in which a dance is filmed. In addition, it is possible to automatically insert effects such as cut change and cross fade depending on types of dance techniques.

In addition, it is possible to, for example, create plug-in software utilizing results of motion recognition for game development environment software, and to apply the plug-in software to game development using movements of the body of a player.

Further, for example, the motion recognition processing of the present technology is applicable to a case where rehabilitation is performed in a hospital or the like.

Specifically, for example, a program and an image indicating a procedure of a correct rehabilitation movement are prepared in advance, and a patient performs the rehabilitation while watching the image. Then, a degree of achievement of the rehabilitation may be detected by recognizing a motion of the patient who is executing the rehabilitation and comparing the movement of the program with the movement of the patient. Then, for example, in a case where the patient is able to execute the rehabilitation in accordance with the procedure of the program, the program may automatically proceed to the next stage. This enables each patient to execute accurate rehabilitation on his or her own in accordance with the program. In addition, it is possible to automatically generate and accumulate an execution record of the rehabilitation of each patient, thus enabling hospital side to automatically manage a progress status of the rehabilitation of each patient.

Further, for example, color and brightness of a pen light or a wristband may be controlled in accordance with motions of audiences who carry or wear the pen light or the wristband whose color, brightness, or the like is variable. This makes it possible to perform rendering or the like in cooperation between a stage and audience seats.

### <Distribution of Motion Data and Rendering Data>

In addition, for example, a professional performer or a choreographer may distribute an image in which a movement (such as choreography or dancing) serving as a model is filmed, and motion data including motion control data corresponding to the movement, via the Internet or the like. It is to be noted that the motion control data is acquired using the motion recognition system of the present technology.

Then, for example, a creator may distribute rendering data for performing rendering in accordance with the movement of the image via the Internet or the like. The rendering data includes, for example, image or sound to be played in accordance with the movement of the image, and control data to be used for controlling of image sound or illumination.

In contrast, for example, a stage director is able to obtain motion data and image data and to produce stage rendering offline using the obtained data. In addition, another performer or user is able to obtain the rendering data and use the rendering data for rendering of a performance to be conducted by each performer or user.

### <Motion to be Recognized>

It is to be noted that the above description has given an example in which the present technology is applied to the case of recognizing the motion of a person; however, the present technology is applicable to a case of recognizing motions of an object other than a person. The object to be recognized may be an organism such as an animal or an inanimate object such as a robot. In a case where the inanimate object is to be recognized, the inanimate object may be an inanimate object, such as a robot, which moves autonomously or by a user operation, or inanimate object such as a sea float that moves by external force such as wave or wind.

### <<8. Modification Example>>

Hereinafter, description is given of a modification example of the above-described embodiments of the techniques according to the present disclosure.

### <Modification Example of Number and Attaching Position of Wearable Sensors>

As described above, it is possible to change the number of the wearable sensors attached and attaching positions (attaching points) thereof in accordance with motions to be recognized, required recognition accuracy, and the like.

For example, in the example of recognizing the dance technique described above, a wearable sensor may be attached to the back, instead of the head and the hip, by using a band or the like.

For example, in a case where the present technology is applied when a user wears AR eyewear and performs a body-based interaction, for example, a wearable sensor is attached to three locations of a head and both wrists. It is to be noted that, instead of attaching the wearable sensor to the head, the AR-eyewear may have a built-in sensor. In addition, a wearable sensor is attached to each of the wrists using, for example, a lightweight band.

For example, in a case where the present technology is applied to command input using the whole body, a wearable sensor is attached to each of four locations of both wrists and both ankles using a lightweight band.

For example, in a case where the motion recognition system is applied to the above-described rehabilitation, the number of the wearable sensors attached and the attaching positions thereof are set in accordance with actions or body locations to be rehabilitated. For example, in a case where an arm is rehabilitated, a wearable sensor is attached to each of two locations of an upper arm and a wrist using a wrap-around type belt. For example, in a case of performing rehabilitation of a walking operation, the wearable sensor is attached to each of four locations of both thighs and both ankles using the wrap-around type belt.

### <Modification Examples of Third Embodiment and Fourth Embodiment>

In addition, the techniques of the third embodiment and the fourth embodiment described above are applicable not only to the communication between the wearable sensor and the motion recognizer but also to communication between a common transmitter and a common receiver.

This makes it possible to immediately start or restart communication without measuring required time or synchronizing time among the transmitter, the relay, and the receiver. In addition, it is possible, in the receiver, to accurately detect time at which a predetermined event is detected.

It is to be noted that there is no particular limitation on an event for which the detection time is to be detected. For example, acceleration, angular velocity, and the like to be detected by each sensor of the wearable sensor are also examples of the event.

Further, even when the communication path between the transmitter and the receiver is dynamically changed, it is possible to immediately start the communication without measuring the required time or synchronizing the time among the transmitter, the relay, and the receiver. In addition, it is possible, in the receiver, to accurately detect time at which the predetermined event is detected.

Further, for example, even when the transmitter delays the transmission of data or thins out the transmission of data for purposes such as power saving, it is possible, in the receiver, to accurately detect the time at which a predetermined event is detected.

It is to be noted that, for example, in a case where the frequency of communication between the transmitter and the receiver is low (e.g., once a day, etc.), when measuring the required time or synchronizing the time among the transmitter, the relay, and the receiver, the cost for measuring the required time or synchronizing the time is larger than the cost for transmitting the actual data. Here, the cost refers to, for example, a cost for securing a communication bandwidth of a communication network, maintenance of a clock, power consumption, or the like. Meanwhile, using the present technology makes it possible to reduce such a waste of cost.

In addition, for example, when detection data is accumulated in a transmitter or an apparatus provided with the transmitter and when the transmitter and the receiver or a relay are ready to be communicable with each other, the present technology is also applicable to a case where the detection data is transmitted from the transmitter to the receiver or the relay. In this case, the transmitter adds the transmission required time to each detection data and transmits the detection data; this transmission required time includes time in which each detection data is accumulated. Accordingly, the transmission required time to be added to each detection data varies depending on the time at which the detection data is detected.

In the following, description is given of a specific example of this application example.

For example, a swimmer performs practices, while wearing transmitters provided with various sensors at a plurality of locations. The transmitter accumulates therein periodically acquired sensor data in a case where the transmitter is unable to communicate with a relay or a receiver, as in a case of underwater. Then, when the transmitter is able to communicate with the receiver, the transmitter transmits each accumulated sensor data together with transmission required time information including accumulation time for each sensor data. Then, for example, the time of occurrence of each event of the swimmer is detected on the basis of the sensor data and the transmission required time. Further, on the basis of the detection result, it is possible to display a graph in which events of the player are arranged in time series, or to animate the form of the swimmer using CG.

For example, a plurality of dancers conduct performances while wearing transmitters provided with various sensors at a plurality of locations. Each transmitter accumulates therein sensor data periodically acquired during the performance, and transmits each sensor data accumulated to a receiver after the performance together with the transmission required time information including the accumulation time for each sensor data. Then, for example, the occurrence time of each event of each dancer is detected on the basis of the sensor data and the transmission required time. On the basis of the detection result, it is possible to confirm timing, accuracy, and the like of the movements of each dancer. Further, it is possible to synchronize and display, for example, the time line of the image in which the performance of each dancer is filmed and the time of occurrence of each event.

For example, sensors provided in a vehicle periodically detect conditions of respective components of the vehicle, and the detected sensor data is accumulated in the vehicle. Then, when the vehicle is parked in a garage, and the transmitter provided in the vehicle and a receiver provided in the garage are ready to be communicable, the transmitter transmits each sensor data accumulated in the vehicle to the receiver together with the transmission required time information including the accumulation time for each sensor data. Then, on the basis of the sensor data and the transmission required time, the time of the occurrence of an event of each component of the vehicle is detected. Further, for example, on the basis of detection results of the event of each component of the vehicle, failure of the component of the vehicle, the time of the occurrence of the failure, the cause of the occurrence, and the like are detected.

For example, a sensor device is attached to each load, and each sensor device periodically detects temperature, humidity, impact, and the like of each load during transportation, and accumulates therein the detected sensor data. Then, in a case where each load arrives at its destination, the sensor device attached to the load transmit each accumulated sensor data to a receiver of the destination together with the transmission required time information including the accumulation time information for each sensor data. Then, on the basis of the sensor data and the transmission required time, an event having occurred to a cargo during transportation
and time of the occurrence thereof are detected.

For example, a sensor device is attached to a patient, and the sensor device periodically detects a vital sign of the patient and accumulates therein the detected sensor data. When the sensor device is brought close to a receiver, the sensor device transmits each accumulated sensor data to the receiver together with the transmission required time information including the accumulation time for each sensor data. Then, on the basis of the sensor data and the transmission required time, each event of the patient and time of the occurrence thereof are detected.

### <Other Modification Examples>

The above-mentioned sharing of functions of respective apparatuses and systems is an exemplary, and may be modified as appropriate. For example, some or all of the functions of the learning processing unit 251 in FIG. 15, the rendering control unit 631 in FIG. 42, or the analysis processing unit 731 in FIG. 47 may be provided in the motion recognizer.

Further, for example, the user may wear the motion recognizer. In this case, the motion recognizer and the wearable sensor may be integrated.

### <<9. Others>>

A series of processing described above may be executed by hardware or software. In a case where the series of processing is executed by software, a program constituting the software is installed in a computer (e.g., the information processor 220). Here, examples of the computer include a computer incorporated in dedicated hardware, and a general-purpose personal computer that is able to execute various functions by installing various programs.

It is to be noted that the program to be executed by the computer may be a program in which processing is performed in time series in the order described in the present specification, or may be a program in which processing is performed in parallel or at a required timing such as when a call is made.

In the present specification, the system means a set of a plurality of components (devices, modules (parts), etc.), and there is no limitation on whether or not all the components are in the same housing. Accordingly, a plurality of devices housed in separate housings and coupled via a network, and a plurality of modules housed in one housing are each a system.

Further, the embodiment of the present technology is not limited to the above-described embodiment, and may be modified in a wide variety of ways without departing from the gist of the present technology.

For example, the present technology may have a configuration of cloud computing in which one function is shared and processed jointly by a plurality of apparatuses via a network.

In addition, each step described in the above-described flowchart may be shared and executed by a plurality of devices, in addition to being performed by one apparatus.

Further, in a case where a plurality of pieces of processing are included in one step, the plurality of pieces of processing included in the one step may be shared and executed by a plurality of devices, in addition to being executed by one apparatus.

### <Examples of Configuration Combinations>

The present technology may have the following configurations.

(1) An information processor including:
   a motion recognition section that recognizes a motion of an object on a basis of a plurality of detection data indicating respective movements of a plurality of detection positions of the object detected by a plurality of sensor apparatuses at the plurality of detection positions; and
   an output control section that controls an output of control data used for controlling of processing corresponding to the motion of the object.
(2) The information processor according to (1), in which
   the object includes a person, and
   the motion includes a unit of movements constituting an action of the person.
(3) The information processor according to (2), in which the detection positions include two or more of a head, a torso, a left hand, a right hand, a left foot, and a right foot of the person.
(4) The information processor according to (2) or (3), further including a rendering control unit that controls rendering of a performance by the person on a basis of the control data.
(5) The information processor according to (4), in which the rendering control unit selects a rendering content on a basis of a combination of a motion before transition and a motion after the transition.
(6) The information processor according to any one of (2) to (5), further including an analysis section that performs an analysis of the action of the person on a basis of the control data.
(7) The information processor according to (6), further including a user interface control section that controls presentation of a result of the analysis of the action of the person.
(8) The information processor according to any one of (1) to (7), in which the motion recognition section recognizes the motion of the object on a basis of a motion relationship between the detection positions.
(9) The information processor according to any one of (1) to (8), further including a reception section that performs near field communication with each of the sensor apparatuses and receives the plurality of detection data.
(10) The information processor according to (9), further including a communication control section that requests the sensor apparatuses to transmit the respective detection data in sequence.
(11) The information processor according to (9) or (10), in which number of retransmissions of the respective detection data between the sensor apparatuses and the information processor is variable.
(12) The information processor according to any one of (1) to (11), in which the control data includes at least one of a result of the motion recognition of the object or control information on the processing corresponding to the motion of the object.
(13) The information processor according to any one of (1) to (12), in which the motion recognition section uses a recognition model for recognition of the motion of the object.
(14) The information processor according to (13), in which the recognition model is learned using learning data in which the respective detection data of the detection positions are partially lost.
(15) The information processor according to (13) or (14), further including a learning section that learns the recognition model.
(16) The information processor according to (15), in which the learning section learns the recognition model by giving weights to the respective detection positions.
(17) The information processor according to any one of (1) to (16), in which the information processor is attached to the object.
(18) The information processor according to (17), including one of the plurality of sensor apparatuses, in which
   the information processor is attached to one of the plurality of detection positions.
(19) An information processing method including causing an information processor to
   recognize a motion of an object on a basis of a plurality of detection data indicating respective movements of a plurality of detection positions of the object detected by a plurality of sensor apparatuses at the plurality of detection positions, and
   control an output of control data used for controlling of processing corresponding to the motion of the object.
(20) A program that causes a computer to execute processing, the processing including:
   a motion recognition step that recognizes a motion of an object on a basis of a plurality of detection data indicating respective movements of a plurality of detection positions of the object detected by a plurality of sensor apparatuses at the plurality of detection positions; and
   an output control step that controls an output of control data used for controlling of processing corresponding to the motion of the object.

### [Reference Numerals List]

- 100: motion recognition system
- 101: wearable sensor
- 102: motion recognizer
- 139: sensor data acquisition section
- 144: packet generation section
- 145: transmission control section
- 161: reception section
- 165: motion recognition section
- 166: transmission control section
- 167: transmission section
- 200: learning system
- 201: learning processor
- 220: information processor
- 221: CPU
- 229: communication unit
- 265: learning data generation section
- 266: learning section
- 267: UI control section
- 300: motion recognition system
- 301: wearable sensor
- 302: motion recognizer
- 331: packet generation section
- 334: transmission control section
- 361: communication control section
- 400: motion recognition system
- 401: wearable sensor
- 402: motion recognizer
- 432: required time estimation section
- 433: packet generation section
- 462: detection time calculation section
- 501: relay
- 531: reception section
- 534: required time estimation section
- 535: packet update section
- 536: transmission control section
- 537: transmission section
- 600: rendering system
- 601: motion recognition system
- 605: rendering controller
- 631: rendering control unit
- 645: scenario generation section
- 646: acoustic control section
- 647: image control section
- 648: illumination control section
- 700: analysis system
- 701: analysis processor
- 731: analysis processing unit
- 741: analysis section
- 742: UI control section

## Claims

1. An information processor comprising:
a motion recognition section that recognizes a motion of an object on a basis of a plurality of detection data indicating respective movements of a plurality of detection positions of the object detected by a plurality of sensor apparatuses at the plurality of detection positions; and
an output control section that controls an output of control data used for controlling of processing corresponding to the motion of the object.

2. The information processor according to claim 1, wherein
the object comprises a person, and
the motion comprises a unit of movements constituting an action of the person.

3. The information processor according to claim 2, wherein the detection positions include two or more of a head, a torso, a left hand, a right hand, a left foot, and a right foot of the person.

4. The information processor according to claim 2, further comprising a rendering control unit that controls rendering of a performance by the person on a basis of the control data.

5. The information processor according to claim 4, wherein the rendering control unit selects a rendering content on a basis of a combination of a motion before transition and a motion after the transition.

6. The information processor according to claim 2, further comprising an analysis section that performs an analysis of the action of the person on a basis of the control data.

7. The information processor according to claim 6, further comprising a user interface control section that controls presentation of a result of the analysis of the action of the person.

8. The information processor according to claim 1, wherein the motion recognition section recognizes the motion of the object on a basis of a motion relationship between the detection positions.

9. The information processor according to claim 1, further comprising a reception section that performs near field communication with each of the sensor apparatuses and receives the plurality of detection data.

10. The information processor according to claim 9, further comprising a communication control section that requests the sensor apparatuses to transmit the respective detection data in sequence.

11. The information processor according to claim 9, wherein number of retransmissions of the respective detection data between the sensor apparatuses and the information processor is variable.

12. The information processor according to claim 1, wherein the control data includes at least one of a result of the motion recognition of the object or control information on the processing corresponding to the motion of the object.

13. The information processor according to claim 1, wherein the motion recognition section uses a recognition model for recognition of the motion of the object.

14. The information processor according to claim 13, wherein the recognition model is learned using learning data in which the respective detection data of the detection positions are partially lost.

15. The information processor according to claim 13, further comprising a learning section that learns the recognition model.

16. The information processor according to claim 15, wherein the learning section learns the recognition model by giving weights to the respective detection positions.

17. The information processor according to claim 1, wherein the information processor is attached to the object.

18. The information processor according to claim 17, comprising one of the plurality of sensor apparatuses, wherein
the information processor is attached to one of the plurality of detection positions.

19. An information processing method comprising causing an information processor to
recognize a motion of an object on a basis of a plurality of detection data indicating respective movements of a plurality of detection positions of the object detected by a plurality of sensor apparatuses at the plurality of detection positions, and
control an output of control data used for controlling of processing corresponding to the motion of the object.

20. A program that causes a computer to execute processing, the processing including:
a motion recognition step that recognizes a motion of an object on a basis of a plurality of detection data indicating respective movements of a plurality of detection positions of the object detected by a plurality of sensor apparatuses at the plurality of detection positions; and
an output control step that controls an output of control data used for controlling of processing corresponding to the motion of the object.
